# EUROPEAN PATENT APPLICATION

(11) **EP 4 740 962 A1**
(43) Date of publication of application: **13.05.2026**
(21) Application number: 24835436.7
(22) Date of filing: 05.07.2024
(51) Int. Cl.: A61K 47/10, A61K 9/16, A61P 25/16

(54) **PHARMACEUTICAL COMPOSITION, FORMULATION CONTAINING PHARMACEUTICAL COMPOSITION, KIT CONTAINING PHARMACEUTICAL COMPOSITION, PREPARATION METHOD FOR PHARMACEUTICAL COMPOSITION, AND USE OF PHARMACEUTICAL COMPOSITION**

(30) Priority: 05.07.2023 CN 202310821933; 02.02.2024 CN 202410157238
(71) Applicant: Shanghai WD Pharmaceutical Co., Ltd, Shanghai 201203 (CN)
(72) Inventor: FAN, Ming, Shanghai 201203 (CN); DONG, Liangchang, Shanghai 201203 (CN); JIAO, Yan, Shanghai 201203 (CN); ZHANG, Danyong, Shanghai 201203 (CN); WANG, Jingyi, Shanghai 201203 (CN); ZHAO, Wenfang, Shanghai 201203 (CN); ZHU, Jianan, Shanghai 201203 (CN); REN, Manman, Shanghai 201203 (CN); WU, Xinyue, Shanghai 201203 (CN)
(74) Representative: Greaves Brewster LLP
(86) International application number: PCT/CN2024/103889
(87) International publication number: WO 2025/007958

(57) **Abstract**

A pharmaceutical composition, a formulation containing the pharmaceutical composition, a kit containing the pharmaceutical composition, a preparation method for the pharmaceutical composition, and a use of the pharmaceutical composition. The pharmaceutical composition comprises the following components: an active pharmaceutical ingredient and a hot-melt adhesive, wherein the mass percentage of the active pharmaceutical ingredient is 0.3%-50%; the hot-melt adhesive is Poloxamer P188 and/or Poloxamer P407; and the mass percentage of the hot-melt adhesive in the pharmaceutical composition is 5%-30%. According to the pharmaceutical composition, the formulation containing the pharmaceutical composition, and the kit containing the pharmaceutical composition, the active pharmaceutical ingredient can be rapidly released to achieve a fast onset of action, the stability of the active pharmaceutical ingredient is improved, the swallowing of patients can be further improved, and the patient compliance during actual medicine-taking can be further improved.

## Description

The present application claims the right of priorities to Chinese Patent Application No. 2023108219336 filed on July 5, 2023 and Chinese Patent Application No. 2024101572389 filed on February 2, 2024. The contents of the above Chinese patent applications are incorporated herein by reference in their entireties.

### TECHNICAL FIELD

The present disclosure relates to a pharmaceutical composition, a formulation containing the pharmaceutical composition, a kit containing the pharmaceutical composition, a preparation method for the pharmaceutical composition, and a use of the pharmaceutical composition.

### BACKGROUND

In certain disease areas, such as motor dysfunction, heart disease, hypertension, asthma, pain, bleeding, bacterial or fungal infections, allergies, influenza, COVID-19, male erectile dysfunction, and gastric reflux, it is required that drugs take effect rapidly after administration to achieve prompt relief and improvement of disease symptoms.

In oral formulations, since solution formulations do not have release issues, they generally take effect relatively quickly after administration. However, solid formulations offer greater advantages for active pharmaceutical ingredients that are moisture-sensitive or have low solubility.

In oral solid formulations, dosage forms such as granules, dispersible tablets, effervescent tablets, orally disintegrating tablets, and oral dissolving films can achieve the characteristic of rapid release of active pharmaceutical ingredients. For pediatric and elderly populations, as well as patients with dysphagia, the aforementioned formulations can facilitate swallowing and improve medication adherence, but they also introduce issues related to palatability and stability.

Specifically in the field of Parkinson's disease, the clinical needs for rapid drug onset and ease of swallowing are described as follows.

Parkinson's disease (PD), also known as paralysis agitans, is one of the most common neurodegenerative diseases, typically onset between the ages of 50 and 65. Epidemiology indicates that there are over 10 million Parkinson's disease patients worldwide, with nearly 3 million in China. In China, more than 100,000 new cases are reported annually, and in recent years, there has been a trend toward younger onset. Parkinson's disease has become the "second most common neurodegenerative disease" after Alzheimer's disease.

Parkinson's disease is a progressive disease caused by the loss of dopamine-producing cells in the brain. Currently, due to the unclear pathogenesis, there are no drugs that can cure the disease or halt its progression; all drugs aim to help control symptoms. In patients with Parkinson's disease, dopamine production in the brain is reduced. Dopamine is an endogenous substance present in the brain and spinal cord, which helps brain nerve cells properly control motor functions. When dopamine levels in the brain decrease, symptoms of Parkinson's disease will occur, such as tremors, muscle stiffness, bradykinesia, or freezing episodes (OFF episodes) like difficulty walking.

Dysphagia is common among patients with Parkinson's disease. An epidemiological study indicates that 11%-81% of Parkinson's patients experience dysphagia, with pulmonary infections caused by swallowing disorders accounting for as high as 91.7% in community statistics (Fatemeh Rajati et al. The global prevalence of oropharyngeal dysphagia in different populations: a systematic review and meta-analysis. Journal of Translational Medicine. 2022, 20:175. https://doi.org/10.1186/s12967-022-03380-0). Dysphagia is a common symptom in Parkinson's patients, causing numerous inconveniences in daily activities such as eating and medication administration, and severely impacting the quality of life, particularly during the "OFF" state.

As Parkinson's disease progresses, when the patient's levodopa concentration decreases, the patient may experience freezing episodes (OFF episodes), characterized by sudden onset and brief duration, during which patients are unable to generate effective forward steps. In some cases, patients may experience complete immobility upon standing, lasting from several seconds to several tens of minutes. Freezing gait is a significant cause of physical injury and disability in Parkinson's patients and increases the financial burden on their families. When patients are driving or crossing the road, the occurrence of freezing episodes can even lead to traffic accidents, resulting in serious consequences. Studies have shown (Li Yan, Wang Lijuan, Zhang Yuhu. Research progress on freezing of gait[J]. Journal of Clinical Neurology. 2016, 29(2): 149-151.) that freezing gait occurs in 7% of patients with early-stage idiopathic Parkinson's disease. Approximately 50% of Parkinson's patients with a disease course exceeding 10 years have experienced freezing gait. As the disease progresses, the frequency and duration of episodes increase.

In summary, designing a drug delivery system that improves dysphagia in patients, enhances medication compliance, and provides rapid onset of action is an effective solution for alleviating Parkinson's freezing episodes. Patients can self-administer the medication when signs of freezing episodes appear or during the early stages of freezing episodes, rapidly increasing the plasma concentration of levodopa, avoiding dangers caused by sudden motor disability, and minimizing disruption to their normal daily life.

Currently available products on the market include APOKYN, KYNMOBI, and INBRIJA. APOKYN is apomorphine hydrochloride injection, which, with a usage frequency of 3-5 times daily, is highly inconvenient for subcutaneous injection. After product upgrade, the injection was changed to a sublingual film formulation (US 10420763 B2), namely KYNMOBI, which is administered sublingually, significantly improving administration compliance. However, KYNMOBI still exhibits relatively high adverse reactions such as nausea. INBRIJA is the first inhaled levodopa product, developed using Acorda's proprietary ARCUS platform (US 8404276 B2), designed to deliver a precise dose of levodopa dry powder formulation to the patient's lungs, thereby avoiding oral swallowing difficulties. However, with INBRIJA, each treatment requires a dose of two capsules; before use, one capsule is required to be loaded into the inhalation device, and after administration, the capsule shell is removed, followed by loading a second capsule for another dose. For Parkinson's patients in the OFF state, this usage procedure is extremely cumbersome. Additionally, this product has a relatively high incidence of adverse reactions (>5%), such as cough, nausea, and upper respiratory tract infections. It is not suitable for patients with lung diseases such as asthma or chronic obstructive pulmonary disease (COPD).

Carbidopa is an aromatic amino acid decarboxylase inhibitor, typically formulated in combination with levodopa (an aromatic amino acid and dopamine prodrug). By inhibiting peripheral decarboxylation of levodopa, carbidopa facilitates more levodopa to be transported to the brain, thereby increasing the half-life and bioavailability of levodopa. Carbidopa is prone to degradation under the influence of external factors, producing degradation products such as DHPA (dihydroxyphenylacetone) and hydrazine. In particular, hydrazine, as a genotoxic substance, should be strictly controlled in the formulation. In commercially available compound formulation products containing levodopa and carbidopa, the content of hydrazine is often high during product storage and use, posing significant safety risks to patients.

Voriconazole is a broad-spectrum triazole antifungal drug, with indications including the treatment of invasive aspergillosis, as well as serious infections caused by Candida, Actinomyces, and Fusarium species, and is one of the most important drugs for treating invasive fungal diseases. Authoritative guidelines in China, such as the "Recommendations for Diagnosis and Treatment of Invasive Fungal Diseases in Children with Hematologic Disorders and Malignant Tumors", recommend voriconazole for the treatment and prevention of invasive aspergillosis in patients.

Currently, oral formulations of voriconazole available both domestically and internationally include tablets, dry suspensions, and capsules. Among these, dry suspensions have the advantage of convenient administration, making them suitable for patients with dysphagia, such as children and the elderly. However, the original drug, voriconazole dry suspension (brand name VFEND^{®}) developed by Pfizer Inc. in the United States, has shortcomings in stability and involves cumbersome usage steps, which may impact patient adherence to medication. The storage condition for this drug is 2-8°C. Before use, the entire bottle of dry suspension is required be reconstituted into a suspension, and the suspension can only be stored for 14 days. The suspension needs to be administered using an oral dosing syringe, which may cause fear of medication in pediatric patients.

Therefore, it is urgent to solve the problem of how to provide a pharmaceutical formulation that facilitates administration, enables rapid release of the active pharmaceutical ingredient, and exhibits good stability. Moreover, it is essential to develop a formulation product containing voriconazole, for example, that offers good stability, ease of use, and improved patient adherence.

### SUMMARY

To address the aforementioned issues in the prior art, the present disclosure provides a pharmaceutical composition, a formulation containing the pharmaceutical composition, a kit containing the pharmaceutical composition, a preparation method for the pharmaceutical composition, and a use of the pharmaceutical composition. The pharmaceutical composition and the formulation and kit thereof not only enable rapid release of the active pharmaceutical ingredient but also exhibit good drug stability, improve patient swallowing, eliminate the need for prior preparation during administration, offer greater convenience in use, and provide significant advantages in terms of medication adherence.

To achieve the above objectives, the technical solutions adopted by the present disclosure are as follows:
A first aspect of the present disclosure provides a pharmaceutical composition comprising the following components: an active pharmaceutical ingredient and a hot-melt adhesive; wherein
the mass percentage of the active pharmaceutical ingredient is 0.3%-63%;
the hot-melt adhesive is:
   Class A hot-melt adhesive: Poloxamer P188 and/or Poloxamer P407;
   or Class B hot-melt adhesive: polyethylene glycol 6000;
   the mass percentage of the Class A hot-melt adhesive in the pharmaceutical composition is 5%-30%;
   the mass percentage of the Class B hot-melt adhesive in the pharmaceutical composition is 12%-25%;
   the pharmaceutical composition is a pharmaceutical composition in the form of a hot-melt granulated granule.

A second aspect of the present disclosure provides a pharmaceutical composition comprising the following components: an active pharmaceutical ingredient and a hot-melt adhesive; wherein
the mass percentage of the active pharmaceutical ingredient is 0.3%-50%;
the hot-melt adhesive is Poloxamer P188 and/or Poloxamer P407;
the mass percentage of the hot-melt adhesive in the pharmaceutical composition is 5%-30%.

For the pharmaceutical composition according to the first aspect and the second aspect, each condition is as described below.

In the present disclosure, the active pharmaceutical ingredient may comprise, but is not limited to, one or more of the following: dopaminergic agents, anti-erectile dysfunction agents, antiviral agents, gastric acid secretion inhibitors, anticoagulants, antifungal agents, antibacterial agents, antiasthmatic agents, antidepressants, antiepileptic agents, antiallergic agents, antihypertensive agents, antipsychotic agents, analgesic agents, anti-inflammatory agents, antiemetic agents, and antitussive agents.

Preferably, the active pharmaceutical ingredient is a poorly soluble and easily degradable drug, a poorly soluble and non-easily degradable drug, a non-poorly soluble and easily degradable drug, or a non-poorly soluble and non-easily degradable drug.

Herein, the poorly soluble and easily degradable drug may be selected from: ritonavir, vonoprazan, rivaroxaban, posaconazole, cefdinir, cefixime, cefuroxime axetil, clarithromycin, linezolid, and duloxetine.

Herein, the poorly soluble and non-easily degradable drug may be selected from: apixaban, tadalafil, dapoxetine, contezolid, olanzapine, aripiprazole, quetiapine fumarate, zolmitriptan, rizatriptan, almotriptan, naratriptan, frovatriptan, eletriptan, and verapamil.

Herein, the poorly soluble and non-easily degradable drug may also be: voriconazole.

Herein, the non-poorly soluble and easily degradable drug may be selected from: carbidopa, oseltamivir phosphate, favipiravir, molnupiravir, deuremidevir hydrobromide, nirmatrelvir, cefaclor, cefprozil, amoxicillin, montelukast sodium, levetiracetam, and desloratadine.

Herein, the non-poorly soluble and non-easily degradable drug may be selected from: levodopa, sildenafil, vardenafil, tegoprazan, fluconazole, escitalopram oxalate, levocetirizine, amlodipine, ketorolac tromethamine, and diphenhydramine.

In the present disclosure, the mass percentage of the active pharmaceutical ingredient in the pharmaceutical composition may be 0.3%-40%.

In the present disclosure, the mass percentage of the hot-melt adhesive in the pharmaceutical composition may be 14%-25%.

In the present disclosure, the mass percentage of the Class A hot-melt adhesive in the pharmaceutical composition may be 14%-25%; and the mass percentage of the Class B hot-melt adhesive in the pharmaceutical composition may be 15.0%-21.6%.

In the present disclosure, the pharmaceutical composition may be a pharmaceutical composition in the form of a hot-melt granulated granule.

In the present disclosure, the pharmaceutical composition may not comprise an effervescent system. Generally, the "effervescent system" refers to a system typically containing an organic acid and sodium carbonate, wherein the system releases carbon dioxide upon contact with an aqueous solution.

In the present disclosure, the pharmaceutical composition may further comprise one or more of the following components: a disintegrant, a filler, a lubricant, a glidant, a flavoring agent, and an aromatic agent.

In the present disclosure, the pharmaceutical composition may further comprise a surfactant.

Herein, the disintegrant may be one or more of croscarmellose sodium, low-substituted hydroxypropyl cellulose, sodium starch glycolate, and crospovidone, preferably croscarmellose sodium and/or low-substituted hydroxypropyl cellulose. The mass percentage of the disintegrant in the pharmaceutical composition may be 0%-30%, preferably 10%-22%.

Herein, the disintegrant may also be microcrystalline cellulose and/or pregelatinized starch.

Herein, the mass percentage of the disintegrant in the pharmaceutical composition may also be 0%-22%.

Herein, the filler may be mannitol and/or lactose, for example, mannitol. The mass percentage of the filler in the pharmaceutical composition may be 0%-50%. In the actual product preparation process, the lactose is generally presented in the form of lactose monohydrate, and those skilled in the art understand that the amount of the filler in the pharmaceutical composition should be adjusted to correspond to the amount of the hydrate form.

Herein, the filler may also be sorbitol.

Herein, the lubricant may be one or more of calcium stearate, glyceryl behenate, magnesium stearate, sodium stearyl fumarate, magnesium silicate, and calcium silicate, such as magnesium stearate. The mass percentage of the lubricant in the pharmaceutical composition may be 0%-2%.

Herein, the glidant may be talc and/or colloidal silica. The mass percentage of the glidant in the pharmaceutical composition may be 0%-2%.

Herein, the flavoring agent may be one or more of sucrose, maltose, stevioside, mannitol, erythrose, and aspartame, preferably one or more of stevioside, mannitol, erythrose, and aspartame. The mass percentage of the flavoring agent in the pharmaceutical composition may be 0%-5%.

Herein, the flavoring agent may also be citric acid.

Herein, the aromatic agent may be a flavor, preferably a natural flavor, such as orange flavor and/or apple flavor. The mass percentage of the aromatic agent in the pharmaceutical composition may be 0%-3%.

Herein, the surfactant may be one or more of glyceryl monostearate, soy lecithin, lecithin, xanthan gum, polyoxyethylene stearate, and sodium dodecyl sulfate. The mass percentage of the surfactant in the pharmaceutical composition may be 0%-15%.

In some embodiments, the active pharmaceutical ingredient is carbidopa and levodopa; the mass percentage of carbidopa in the pharmaceutical composition is 0.5%-15%; the mass percentage of levodopa in the pharmaceutical composition is 2%-48%; the mass ratio of carbidopa to levodopa is 1:1-1:24. In the actual product preparation process, the carbidopa is generally presented in the form of a monohydrate, and those skilled in the art understand that the amount of the carbidopa in the pharmaceutical composition should be adjusted to correspond to the amount of the hydrate form.

In some preferred embodiments, the pharmaceutical composition comprises the following components in percentage by mass: 0.5%-10% carbidopa, 2%-40% levodopa, 6%-30% hot-melt adhesive, and 0-30% disintegrant.

In some preferred embodiments, the pharmaceutical composition comprises the following components in percentage by mass: 0.5%-10% carbidopa, 2%-40% levodopa, 6%-30% Class A hot-melt adhesive, and 0-30% disintegrant.

In some preferred embodiments, the pharmaceutical composition comprises the following components in percentage by mass: 5.7%-8.3% carbidopa, 23%-33% levodopa, 15%-25% hot-melt adhesive, 10.1%-20.1% low-substituted hydroxypropyl cellulose, and 23.2%-33.2% mannitol; or, the pharmaceutical composition comprises 6.5%-7.5% carbidopa, 25.1%-30.1% levodopa, 17.5%-22.5% hot-melt adhesive, 13.5%-18.5% low-substituted hydroxypropyl cellulose, and 27.0%-32.0% mannitol.

In one specific embodiment, the pharmaceutical composition comprises: 7.5% carbidopa monohydrate, 27.6% levodopa, 21.6% Poloxamer 407, 15.1% low-substituted hydroxypropyl cellulose, and 28.2% mannitol.

In one specific embodiment, the pharmaceutical composition comprises: 7.5% carbidopa monohydrate, 27.6% levodopa, 18.5% Poloxamer 407, 15.1% low-substituted hydroxypropyl cellulose, and 31.3% mannitol.

In one specific embodiment, the pharmaceutical composition comprises: 10.6% carbidopa monohydrate, 39.3% levodopa, 13% Poloxamer 407, 10% low-substituted hydroxypropyl cellulose, and 27.1% mannitol.

In one specific embodiment, the pharmaceutical composition comprises: 10.5% carbidopa monohydrate, 39% levodopa, 12.8% Poloxamer 407, 9.9% low-substituted hydroxypropyl cellulose, 26.8% mannitol, 0.5% magnesium stearate, and 0.5% orange flavor.

In one specific embodiment, the pharmaceutical composition comprises: 2.7% carbidopa monohydrate, 10% levodopa, 23% Poloxamer 407, 14% low-substituted hydroxypropyl cellulose, 49.3% lactose, 0.5% colloidal silica, and 0.5% aspartame.

In one specific embodiment, the pharmaceutical composition comprises: 3.2% carbidopa monohydrate, 12% levodopa, 20% Poloxamer 407, 18% sodium carboxymethyl starch, 45.9% lactose, 0.6% magnesium stearate, and 0.3% orange flavor.

In one specific embodiment, the pharmaceutical composition comprises: 4.4% carbidopa monohydrate, 16.4% levodopa, 6.5% Poloxamer 407, 17.3% crospovidone, 49.6% mannitol, 0.8% colloidal silica, 4.7% sucrose, and 0.3% apple flavor.

In one specific embodiment, the pharmaceutical composition comprises: 6.5% carbidopa monohydrate, 24% levodopa, 20.6% Poloxamer 407, 15% lactose, and 33.9% mannitol.

In one specific embodiment, the pharmaceutical composition comprises: 7.5% carbidopa monohydrate, 27.6% levodopa, 29.9% Poloxamer 407, 29% croscarmellose sodium, 1% magnesium stearate, and 5% sucrose.

In one specific embodiment, the pharmaceutical composition comprises: 0.675% carbidopa monohydrate, 2.5% levodopa, 25% Poloxamer 407, 22.5% croscarmellose sodium, and 49.325% mannitol.

In one specific embodiment, the pharmaceutical composition comprises: 8.4% carbidopa monohydrate, 31.2% levodopa, 16.5% Poloxamer 407, 11.5% croscarmellose sodium, and 32.4% mannitol.

In one specific embodiment, the pharmaceutical composition comprises: 9.7% carbidopa monohydrate, 36% levodopa, 18% Poloxamer 407, 12% low-substituted hydroxypropyl cellulose, and 24.3% mannitol.

In one specific embodiment, the pharmaceutical composition comprises: 10.4% carbidopa monohydrate, 38.4% levodopa, 15% Poloxamer 407, 18% low-substituted hydroxypropyl cellulose, and 18.2% lactose.

In one specific embodiment, the pharmaceutical composition comprises: 10.4% carbidopa monohydrate, 38.4% levodopa, 16% Poloxamer 407, 12% low-substituted hydroxypropyl cellulose, and 23.2% mannitol.

In one specific embodiment, the pharmaceutical composition comprises: 10.4% carbidopa monohydrate, 38.4% levodopa, 23% Poloxamer 407, 5% low-substituted hydroxypropyl cellulose, and 23.2% mannitol.

In one specific embodiment, the pharmaceutical composition comprises: 7.5% carbidopa monohydrate, 27.6% levodopa, 18.5% Poloxamer 407, 13% low-substituted hydroxypropyl cellulose, and 33.4% mannitol.

In one specific embodiment, the pharmaceutical composition comprises: 2.5% apixaban, 25% Poloxamer 188, 20% croscarmellose sodium, and 52.5% lactose monohydrate.

In one specific embodiment, the pharmaceutical composition comprises: 2.5% apixaban, 18% Poloxamer 407, 15% croscarmellose sodium, and 64.5% lactose monohydrate.

A third aspect of the present disclosure provides a pharmaceutical composition comprising an active pharmaceutical ingredient and a hot-melt adhesive; wherein the active pharmaceutical ingredient is voriconazole; the hot-melt adhesive is selected from:
Class A hot-melt adhesive: Poloxamer P188 and/or Poloxamer P407;
and Class B hot-melt adhesive: polyethylene glycol 6000;
the mass percentage of the Class B hot-melt adhesive in the pharmaceutical composition is 12%-25%;
the pharmaceutical composition is a pharmaceutical composition in the form of a hot-melt granulated granule.

A fourth aspect of the present disclosure provides a pharmaceutical composition comprising an active pharmaceutical ingredient and a hot-melt adhesive; wherein the active pharmaceutical ingredient is voriconazole; the hot-melt adhesive is:
Class A hot-melt adhesive: Poloxamer P188 and/or Poloxamer P407;
or Class B hot-melt adhesive: polyethylene glycol 6000;
the mass percentage of the Class B hot-melt adhesive in the pharmaceutical composition is 12%-25%;
the pharmaceutical composition is a pharmaceutical composition in the form of a hot-melt granulated granule.

For the pharmaceutical composition according to third aspect and the fourth aspect, each condition is as described below.

In the present disclosure, the mass percentage of the active pharmaceutical ingredient in the pharmaceutical composition may be 1%-63%, preferably 8.3%-63%.

In the present disclosure, the mass percentage of the Class A hot-melt adhesive in the pharmaceutical composition may be 5%-30%, preferably 12%-25%, and more preferably 15.0%-21.6%.

In the present disclosure, the mass percentage of the Class B hot-melt adhesive in the pharmaceutical composition is preferably 15.0%-21.6%.

In the present disclosure, the pharmaceutical composition may further comprise one or more of the following components: a disintegrant, a filler, a lubricant, a glidant, and a flavoring agent.

In the present disclosure, the pharmaceutical composition may further comprise a surfactant.

Herein, the disintegrant may be one or more of croscarmellose sodium, low-substituted hydroxypropyl cellulose, sodium starch glycolate, microcrystalline cellulose, pregelatinized starch, and crospovidone, preferably croscarmellose sodium and/or low-substituted hydroxypropyl cellulose. The mass percentage of the disintegrant in the pharmaceutical composition may be 0%-30%, preferably 0%-20%.

Herein, the filler may be one or more of mannitol, sorbitol, and lactose, such as mannitol. The mass percentage of the filler in the pharmaceutical composition may be 0%-50%.

Herein, the lubricant may be one or more of calcium stearate, glyceryl behenate, magnesium stearate, sodium stearyl fumarate, magnesium silicate, and calcium silicate, such as magnesium stearate. The mass percentage of the lubricant in the pharmaceutical composition may be 0%-2%.

Herein, the glidant may be talc and/or colloidal silica. The mass percentage of the glidant in the pharmaceutical composition may be 0%-2%.

Herein, the flavoring agent may be one or more of sucrose, maltose, stevioside, mannitol, erythrose, aspartame, citric acid, and flavor, preferably one or more of stevioside, mannitol, erythrose, aspartame, and flavor. The mass percentage of the flavoring agent in the pharmaceutical composition may be 0%-5%.

Herein, the surfactant may be one or more of glyceryl monostearate, soy lecithin, lecithin, xanthan gum, polyoxyethylene stearate, and sodium dodecyl sulfate. The mass percentage of the surfactant in the pharmaceutical composition may be 0%-15%.

In some embodiments, the pharmaceutical composition comprises the following components in percentage by mass: 1%-63% of the active pharmaceutical ingredient, "5%-30% of the Class A hot-melt adhesive or 12%-25% of the Class B hot-melt adhesive", 0%-30% of the disintegrant, 0%-50% of the filler, 0%-2% of the lubricant, 0%-2% of the glidant, and 0%-5% of the flavoring agent.

In some embodiments, the pharmaceutical composition comprises the following components in percentage by mass: 1%-63% of the active pharmaceutical ingredient, 5%-30% of "the Class A hot-melt adhesive and the Class B hot-melt adhesive", 0%-30% of the disintegrant, 0%-50% of the filler, 0%-2% of the lubricant, 0%-2% of the glidant, and 0%-5% of the flavoring agent.

In some embodiments, the pharmaceutical composition comprises the following components in percentage by mass: 8.3%-63% of voriconazole, 12%-25% of the hot-melt adhesive, 0%-20% of the disintegrant, 0%-50% of the filler, 0%-2% of the lubricant, 0%-2% of the glidant, and 0%-5% of the flavoring agent.

In some embodiments, the pharmaceutical composition comprises the following components in percentage by mass: 33%-55% of voriconazole, 15.0%-21.6% of the hot-melt adhesive, 5-18% of the disintegrant, 8.5%-36.5% of the filler, 0%-1% of the lubricant, 0.5%-1.5% of the glidant, and 0.5%-1.5% of the flavoring agent.

A fifth aspect of the present disclosure provides a pharmaceutical formulation, which is a hot-melt granulated granule, and the pharmaceutical formulation comprises the pharmaceutical composition as described above (first aspect to fourth aspect).

Herein, the particle size of the hot-melt granulated granule may be less than 1000 µm.

A sixth aspect of the present disclosure provides an oral administration delivery formulation kit, wherein the oral administration delivery formulation kit comprises an oral administration delivery device AcuSiS^{®} and the pharmaceutical formulation as described above (the fifth aspect); the pharmaceutical formulation is disposed in a drug-carrying space of the oral administration delivery device AcuSiS^{®}.

In the present disclosure, the oral administration delivery formulation kit does not require pre-preparation before use; its lower end is immersed in water or a beverage and actively sucked by the patient, making it suitable for patients with dysphagia such as children and the elderly; the pharmaceutical formulation filled in the oral administration delivery device AcuSiS^{®} exhibits higher stability, facilitating convenient storage and use. This oral delivery device can significantly alleviate dysphagia in patients, particularly improving dysphagia in Parkinson's patients during the "off" state. It does not require pre-preparation before use, and the pharmaceutical composition it carries exhibits faster dissolution rates, which is of great significance for effectively relieving the "off" state in Parkinson's patients. The hot-melt granulated granules filled in the oral administration delivery device AcuSiS^{®} exhibit higher stability and can reduce the degradation product DHPA and the genotoxic impurity hydrazine generated during storage of the formulation.

Preferably, the filling amount of the pharmaceutical composition is 50-1000 mg.

Preferably, the pharmaceutical formulation is a carbidopa-levodopa granule comprising 25 mg of carbidopa, 100 mg of levodopa, 66.9 mg of a hot-melt adhesive, 54.6 mg of low-substituted hydroxypropyl cellulose, and 113.3 mg of mannitol.

Herein, the oral administration delivery device AcuSiS^{®} is a commercially available oral administration delivery device, which may be, for example, the oral administration delivery device protected in the Chinese Utility Model Patent CN201921652286.6, and any product from the Examples 1-9 described therein can be used in the present disclosure; meanwhile, similar devices capable of achieving the same effect as the oral administration delivery device may also be used. Generally, the oral administration delivery device may comprise five components, namely a high-density polyethylene (HDPE) pharmaceutical AcuSiS^{®} cap, a polypropylene (PP) pharmaceutical AcuSiS^{®} straw, a high-density polyethylene (HDPE) pharmaceutical AcuSiS^{®} filter housing body, a polypropylene (PP) melt-blown nonwoven fabric disc, and a high-density polyethylene (HDPE) pharmaceutical AcuSiS^{®} filter housing cap bottom piece.

Herein, the high-density polyethylene (HDPE) pharmaceutical AcuSiS^{®} filter housing body, the polypropylene (PP) melt-blown nonwoven fabric disc, and the high-density polyethylene (HDPE) pharmaceutical AcuSiS^{®} filter housing cap bottom piece are assembled to form an AcuSiS^{®} filter; the polypropylene (PP) pharmaceutical AcuSiS^{®} straw and the AcuSiS^{®} filter are assembled to form an AcuSiS^{®} body; the granules are filled and stored in the AcuSiS^{®} body and sealed with a high-density polyethylene (HDPE) pharmaceutical AcuSiS^{®} cap.

Herein, each said oral administration delivery formulation kit may further comprise an encapsulation layer for sealing the oral administration delivery device AcuSiS^{®}.

Preferably, the encapsulation layer is a polyester-aluminum-polyethylene composite film bag.

Preferably, a desiccant is further provided in the encapsulation layer, wherein the desiccant is a bagged desiccant, more preferably a 0.25 g bagged desiccant.

A seventh aspect of the present disclosure further provides a use of the pharmaceutical composition as described above (the second aspect), wherein the active pharmaceutical ingredients are carbidopa and levodopa, in the manufacture of a medicament for emergency or intermittent treatment of a Parkinson's off episode.

An eighth aspect of the present disclosure further provides a use of the pharmaceutical composition as described above (the fourth aspect), wherein the active pharmaceutical ingredient is voriconazole, in the manufacture of a medicament for the treatment or prevention of invasive fungal disease.

A ninth aspect of the present disclosure further provides a preparation method for the pharmaceutical formulation as described above (the fifth aspect), wherein the preparation method is a hot-melt granulation method comprising the following steps:
pulverizing the hot-melt adhesive to obtain a powder, mixing with the active pharmaceutical ingredient, a disintegrant, and a filler (when no disintegrant and filler are included, they are not added), performing hot-melt granulation, then uniformly mixing with the remaining components of the pharmaceutical composition, and sieving to obtain the pharmaceutical formulation;
alternatively, for the pharmaceutical formulation of the pharmaceutical composition according to the aforementioned fourth aspect, pulverizing the hot-melt adhesive to obtain a powder, mixing with the active pharmaceutical ingredient, a disintegrant, a filler, a lubricant, and a glidant, performing hot-melt granulation, then uniformly mixing with the remaining components of the pharmaceutical composition, and sizing and sieving to obtain the pharmaceutical formulation.

In the present disclosure, the pharmaceutical formulation prepared by the hot-melt granulation method can significantly improve the compatibility of each component of the pharmaceutical composition, further promote rapid release, enhance stability, and reduce the generation of DHPA and the genotoxic impurity hydrazine during the production process.

Herein, the particle size of the powder may be less than 40 mesh.

Herein, the temperature for the hot-melt granulation may be 50-100°C, preferably 55-80°C, such as 60°C, 70°C, or 80°C.

For example, the preparation method comprises the following steps: performing hot-melt granulation using a twin-screw extruder, with a standard configuration of screw assembly, a granulation temperature of 60-90°C, preferably 60-70°C, a screw speed of 200 rpm, and a feed speed of 30 rpm, and passing the obtained granules through a 20-mesh sieve to obtain product granules; or, performing granulation in a high-shear granulator with a heating jacket at a granulation temperature of 70-80°C until uniform granules are formed, and passing the hot-melt granules through a 20-mesh sieve to obtain product granules; or, mixing in a square cone mixer at a speed of 10 rpm for 10 minutes to obtain product granules.

For example, for the pharmaceutical formulation of the pharmaceutical composition according to the aforementioned fourth aspect, the preparation method comprises the following steps: performing hot-melt granulation using a twin-screw extruder at a granulation temperature of 55-90°C, preferably 60-80°C, with a screw speed of 100-800 rpm, and a feed rate of 5-80%, and sizing by passing the obtained granules through a 14- or 16-mesh sieve to obtain product granules; or, performing granulation in a high-shear granulator with a heating jacket at a granulation temperature of 70-80°C until uniform granules are formed, and passing the hot-melt granules through a 14- or 16-mesh sieve to obtain product granules; or, mixing the granules after hot-melt granulation with the remaining components of the pharmaceutical composition using a square cone mixer at a speed of 10 rpm for 5-30 min to obtain product granules.

On the basis of common knowledge in the art, the preferred conditions above can be arbitrarily combined to obtain the preferred examples of the present disclosure.

The reagents and raw materials used in the present disclosure are commercially available.

The positive and progressive effects of the present disclosure are as follows:
The present disclosure uses a specific amount of a particular adhesive in combination with a specific amount of an active pharmaceutical ingredient, employing a specific hot-melt granulation process to produce a pharmaceutical composition. The resulting pharmaceutical composition enables rapid release of the active pharmaceutical ingredient, ensures good raw material compatibility, and improves the stability of the active pharmaceutical ingredient. Particularly for products where the active pharmaceutical ingredients are carbidopa and levodopa, it is particularly suitable for improving the rapid release and stability of the active pharmaceutical ingredients used in emergency or intermittent treatment of Parkinson's "off" periods, as well as for improving the stability of voriconazole dry suspension for pediatric use. The resulting pharmaceutical composition, administered via a specific oral administration delivery device, does not require prior preparation and is actively sucked by the patient, significantly improving swallowing in patients with dysphagia, thereby enhancing patient adherence and therapeutic outcomes.

### BRIEF DESCRIPTION OF THE DRAWINGS

FIG. 1 shows the product appearance of the oral administration delivery formulation kit of the present disclosure.
   1-oral administration delivery device AcuSiS^{®}, 2-drug granules.
FIG. 2 shows a schematic diagram of the use of the oral administration delivery formulation kit of the present disclosure, where a of FIG. 2 shows a schematic diagram of the oral administration delivery formulation kit immediately after insertion into water, and b of FIG. 2 shows a schematic diagram of granules in the oral administration delivery formulation kit being sucked through a straw.
FIG. 3 shows the drug-time curve of levodopa from the carbidopa-levodopa granule formulation in Effect Example 4 of the present disclosure and a commercially available carbidopa-levodopa tablet.
FIG. 4 shows a partially enlarged drug-time curve of levodopa from the carbidopa-levodopa granule formulation in Effect Example 4 of the present disclosure and a commercially available carbidopa-levodopa tablet as shown in FIG. 3.

### DETAILED DESCRIPTION OF THE PREFERRED EMBODIMENT

The present disclosure is further described below by the way of examples, but the present disclosure is not thereby limited to the scope of the described examples. The experimental methods for which the specific conditions are not specified in the following examples are selected according to the conventional methods and conditions, or according to the commodity instructions.

### Example 1

According to the formulation in Table 1, carbidopa-levodopa granules were prepared by the following hot-melt granulation process:

Poloxamer 407 was pulverized and passed through an 80-mesh sieve to obtain Poloxamer 407 powder.

Carbidopa, levodopa, and other excipients were added to a square cone mixer and mixed at a speed of 10 rpm for 10 minutes, and the mixture was discharged.

The resulting mixture was placed in a high-shear granulator with a heating jacket for granulation at a temperature of 70-80°C until uniform granules were formed. The hot-melt granules were passed through a 20-mesh sieve to obtain carbidopa-levodopa granules.

**Table 1. Specific component information of Example 1**

| Component | mg per unit | wt% |
|---|---|---|
| Carbidopa monohydrate | 27.0* | 7.5 |
| Levodopa | 100.0 | 27.6 |
| Mannitol | 102.2 | 28.2 |
| Poloxamer 407 | 78.1 | 21.6 |
| Low-substituted hydroxypropyl cellulose | 54.5 | 15.1 |
| Total | 361.8 | 100.0 |

| | | |
|---|---|---|
| Note: * indicates the amount of carbidopa monohydrate, equivalent to 25 mg (6.9%) of carbidopa. | | |

### Example 2

According to the formulation in Table 2, carbidopa-levodopa granules were prepared by the following hot-melt granulation process:
Poloxamer 407 was pulverized and passed through a 40-mesh sieve to obtain Poloxamer 407 powder.
Carbidopa, levodopa active pharmaceutical ingredients and excipients were added to a square cone mixer and mixed at a speed of 10 rpm for 10 minutes, and the mixture was discharged.

Hot-melt granulation was carried out using a twin-screw extruder. The screw assembly adopted a standard configuration, with a granulation temperature of 60-70°C, a screw speed of 200 rpm, and a feed speed of 30 rpm. The obtained granules were passed through a 20-mesh sieve to obtain carbidopa-levodopa granules.

**Table 2. Specific component information of Example 2**

| Component | mg per unit | wt% |
|---|---|---|
| Carbidopa monohydrate | 27.0* | 7.5 |
| Levodopa | 100.0 | 27.6 |
| Mannitol | 113.3 | 31.3 |
| Poloxamer 407 | 66.9 | 18.5 |
| Low-substituted hydroxypropyl cellulose | 54.6 | 15.1 |
| Total | 361.8 | 100.0 |

| | | |
|---|---|---|
| Note: * indicates the amount of carbidopa monohydrate, equivalent to 25 mg (6.9%) of carbidopa. | | |

### Example 3

According to the formulation in Table 3, carbidopa-levodopa granules were prepared by the hot-melt granulation process of Example 2.

**Table 3. Specific component information of Example 3**

| Component | mg per unit | wt% |
|---|---|---|
| Carbidopa monohydrate | 27.0* | 10.6 |
| Levodopa | 100.0 | 39.3 |
| Mannitol | 68.8 | 27.1 |
| Poloxamer 407 | 33.0 | 13.0 |
| Low-substituted hydroxypropyl cellulose | 25.4 | 10.0 |
| Total | 254.2 | 100.0 |

| | | |
|---|---|---|
| Note: * indicates the amount of carbidopa monohydrate, equivalent to 25 mg (9.8%) of carbidopa. | | |

### Example 4

According to the formulation in Table 4, the granules from Example 3 were added with 0.5% magnesium stearate and 0.5% natural orange flavor, then placed in a square cone mixer, and mixed at a speed of 10 rpm for 10 minutes to obtain carbidopa-levodopa granules.

**Table 4. Specific component information of Example 4**

| Component | mg per unit | wt% |
|---|---|---|
| Carbidopa monohydrate | 27.0* | 10.5 |
| Levodopa | 100.0 | 39.0 |
| Mannitol | 68.8 | 26.8 |
| Poloxamer 407 | 33.0 | 12.8 |
| Low-substituted hydroxypropyl cellulose | 25.4 | 9.9 |
| Magnesium stearate | 1.28 | 0.5 |
| Natural orange flavor | 1.28 | 0.5 |
| Total | 256.7 | 100.0 |

| | | |
|---|---|---|
| Note: * indicates the amount of carbidopa monohydrate, equivalent to 25 mg (9.7%) of carbidopa. | | |

### Example 5

According to the formulation in Table 5, carbidopa-levodopa granules were prepared by the following hot-melt granulation process:
Poloxamer 407 was pulverized and passed through a 40-mesh sieve to obtain Poloxamer 407 powder.
Carbidopa, levodopa, and other excipients were added to a square cone mixer and mixed at a speed of 10 rpm for 10 minutes, and the mixture was discharged.

Hot-melt granulation was carried out using a twin-screw extruder. The screw assembly adopted a standard configuration, with a granulation temperature of 70-90°C, a screw speed of 200 rpm, and a feed speed of 10 rpm. The obtained granules were passed through a 20-mesh sieve to obtain carbidopa-levodopa granules.

**Table 5. Specific component information of Example 5**

| Component | mg per unit | wt% |
|---|---|---|
| Carbidopa monohydrate | 27.0* | 2.70 |
| Levodopa | 100.0 | 10.0 |
| Lactose | 493.0 | 49.3 |
| Poloxamer 407 | 230.0 | 23.0 |
| Low-substituted hydroxypropyl cellulose | 140.0 | 14.0 |
| Aspartame | 5.0 | 0.5 |
| Colloidal silica | 5.0 | 0.5 |
| Total | 1000.0 | 100.0 |

| | | |
|---|---|---|
| Note: * indicates the amount of carbidopa monohydrate, equivalent to 25 mg (2.5%) of carbidopa. | | |

### Example 6

According to the formulation in Table 6, carbidopa-levodopa granules were prepared by the hot-melt granulation process of Example 5.

**Table 6. Specific component information of Example 6**

| Component | mg per unit | wt% |
|---|---|---|
| Carbidopa monohydrate | 27.0* | 3.2 |
| Levodopa | 100.0 | 12.0 |
| Lactose | 383.0 | 45.9 |
| Poloxamer 407 | 166.6 | 20.0 |
| Sodium carboxymethyl starch | 150.0 | 18.0 |
| Orange flavor | 2.1 | 0.3 |
| Magnesium stearate | 5.0 | 0.6 |
| Total | 833.7 | 100.0 |

| | | |
|---|---|---|
| Note: * indicates the amount of carbidopa monohydrate, equivalent to 25 mg (3.0%) of carbidopa. | | |

### Example 7

According to the formulation in Table 7, carbidopa-levodopa granules were prepared by the hot-melt granulation process of Example 5.

**Table 7. Specific component information of Example 7**

| Component | mg per unit | wt% |
|---|---|---|
| Carbidopa monohydrate | 27.0* | 4.4 |
| Levodopa | 100.0 | 16.4 |
| Mannitol | 302.4 | 49.6 |
| Poloxamer 407 | 39.6 | 6.5 |
| Sucrose | 28.7 | 4.7 |
| Crospovidone | 105.5 | 17.3 |
| Apple flavor | 1.8 | 0.3 |
| Colloidal silica | 4.9 | 0.8 |
| Total | 609.9 | 100.0 |

| | | |
|---|---|---|
| Note: * indicates the amount of carbidopa monohydrate, equivalent to 25 mg (4.1%) of carbidopa. | | |

### Example 8

According to the formulation in Table 8, carbidopa-levodopa granules were prepared by the hot-melt granulation process of Example 5.

**Table 8. Specific component information of Example 8**

| Component | mg per unit | wt% |
|---|---|---|
| Carbidopa monohydrate | 27.0* | 6.5 |
| Levodopa | 100.0 | 24.0 |
| Lactose | 62.5 | 15.0 |
| Mannitol | 141.1 | 33.9 |
| Poloxamer 407 | 86.0 | 20.6 |
| Total | 416.6 | 100.0 |

| | | |
|---|---|---|
| Note: * indicates the amount of carbidopa monohydrate, equivalent to 25 mg (6.0%) of carbidopa. | | |

### Example 9

According to the formulation in Table 9, carbidopa-levodopa granules were prepared by the hot-melt granulation process of Example 5.

**Table 9. Specific component information of Example 9**

| Component | mg per unit | wt% |
|---|---|---|
| Carbidopa monohydrate | 27.0* | 7.5 |
| Levodopa | 100.0 | 27.6 |
| Poloxamer 407 | 108.2 | 29.9 |
| Sucrose | 18.0 | 5.0 |
| Croscarmellose sodium | 105.0 | 29.0 |
| Magnesium stearate | 3.6 | 1.0 |
| Total | 361.8 | 100.0 |

| | | |
|---|---|---|
| Note: * indicates the amount of carbidopa monohydrate, equivalent to 25 mg (6.9%) of carbidopa. | | |

### Example 10

According to the formulation in Table 10, carbidopa-levodopa granules were prepared by the hot-melt granulation process of Example 5.

**Table 10. Specific component information of Example 5**

| Component | mg per unit | wt% |
|---|---|---|
| Carbidopa monohydrate | 13.5* | 0.675 |
| Levodopa | 50.0 | 2.5 |
| Mannitol | 986.5 | 49.325 |
| Poloxamer 407 | 500.0 | 25.0 |
| Croscarmellose sodium | 450.0 | 22.5 |
| Total | 2000.0 | 100.0 |

| | | |
|---|---|---|
| Note: * indicates the amount of carbidopa monohydrate, equivalent to 12.5 mg (0.625%) of carbidopa. | | |

### Example 11

According to the formulation in Table 11, carbidopa-levodopa granules were prepared by the hot-melt granulation process of Example 5.

**Table 11. Specific component information of Example 11**

| Component | mg per unit | wt% |
|---|---|---|
| Carbidopa monohydrate | 27.0* | 8.4 |
| Levodopa | 100.0 | 31.2 |
| Mannitol | 103.8 | 32.4 |
| Poloxamer 407 | 52.9 | 16.5 |
| Croscarmellose sodium | 36.9 | 11.5 |
| Total | 320.6 | 100.0 |

| | | |
|---|---|---|
| Note: * indicates the amount of carbidopa monohydrate, equivalent to 25 mg (7.8%) of carbidopa. | | |

### Example 12

According to the formulation in Table 12, carbidopa-levodopa granules were prepared by the hot-melt granulation process of Example 5.

**Table 12. Specific component information of Example 5**

| Component | mg per unit | wt% |
|---|---|---|
| Carbidopa monohydrate | 27.0* | 9.7 |
| Levodopa | 100.0 | 36.0 |
| Mannitol | 67.5 | 24.3 |
| Poloxamer 407 | 50.0 | 18.0 |
| Low-substituted hydroxypropyl cellulose | 33.3 | 12.0 |
| Total | 277.8 | 100.0 |

| | | |
|---|---|---|
| Note: * indicates the amount of carbidopa monohydrate, equivalent to 25 mg (9.0%) of carbidopa. | | |

### Example 13

According to the formulation in Table 13, carbidopa-levodopa granules were prepared by the hot-melt granulation process of Example 5.

**Table 13. Specific component information of Example 13**

| Component | mg per unit | wt% |
|---|---|---|
| Carbidopa monohydrate | 27.0* | 10.4 |
| Levodopa | 100.0 | 38.4 |
| Lactose | 47.4 | 18.2 |
| Poloxamer 407 | 39.0 | 15.0 |
| Low-substituted hydroxypropyl cellulose | 46.8 | 18.0 |
| Total | 260.2 | 100.0 |

| | | |
|---|---|---|
| Note: * indicates the amount of carbidopa monohydrate, equivalent to 25 mg (9.6%) of carbidopa. | | |

### Example 14

According to the formulation in Table 14, carbidopa-levodopa granules were prepared by the hot-melt granulation process of Example 5.

**Table 14. Specific component information of Example 14**

| Component | mg per unit | wt% |
|---|---|---|
| Carbidopa monohydrate | 27.0* | 10.4 |
| Levodopa | 100.0 | 38.4 |
| Mannitol | 60.4 | 23.2 |
| Poloxamer 407 | 41.6 | 16.0 |
| Low-substituted hydroxypropyl cellulose | 31.2 | 12.0 |
| Total | 260.2 | 100.0 |

| | | |
|---|---|---|
| Note: * indicates the amount of carbidopa monohydrate, equivalent to 25 mg (9.6%) of carbidopa. | | |

### Example 15

According to the formulation in Table 15, carbidopa-levodopa granules were prepared by the hot-melt granulation process of Example 5.

**Table 15. Specific component information of Example 15**

| Component | mg per unit | wt% |
|---|---|---|
| Carbidopa monohydrate | 27.0* | 10.4 |
| Levodopa | 100.0 | 38.4 |
| Mannitol | 60.4 | 23.2 |
| Poloxamer 407 | 59.8 | 23.0 |
| Low-substituted hydroxypropyl cellulose | 13.0 | 5.00 |
| Total | 260.2 | 100.0 |

| | | |
|---|---|---|
| Note: * indicates the amount of carbidopa monohydrate, equivalent to 25 mg (9.6%) of carbidopa. | | |

### Example 16

According to the formulation in Table 16, apixaban granules were prepared by the following hot-melt granulation process:
Poloxamer 188 was pulverized and passed through an 80-mesh sieve to obtain Poloxamer 188 powder.

Excipients, other than apixaban, were added to a square cone mixer and mixed at a speed of 10 rpm for 5 minutes, then discharged. Then, the API and the remaining excipients were mixed by incremental addition in a ratio of 1:2 (i.e., apixaban (or apixaban-containing material) to excipients). After each addition of excipients, mixing was performed at a speed of 10 rpm for 5 minutes. After the final addition of excipients, mixing was performed at a speed of 10 rpm for 15 minutes.

Hot-melt granulation was carried out using a twin-screw extruder. The screw assembly adopted a standard configuration, with a granulation temperature of 70-80°C, a screw speed of 400 rpm, and a feed speed of 40 rpm. The obtained granules were passed through a 20-mesh sieve to obtain apixaban granules.

**Table 16. Specific component information of Example 16**

| Component | mg per unit | wt% |
|---|---|---|
| Apixaban | 2.5 | 2.5 |
| Lactose monohydrate | 52.5 | 52.5 |
| Poloxamer 188 | 25.0 | 25.0 |
| Croscarmellose sodium | 20.0 | 20.0 |
| Total | 100.0 | 100.0 |

### Example 17

According to the formulation in Table 17, apixaban granules were prepared by the following hot-melt granulation process:
Poloxamer 407 was pulverized and passed through an 80-mesh sieve to obtain Poloxamer 407 powder.

Excipients, other than apixaban, were added to a square cone mixer and mixed at a speed of 10 rpm for 5 minutes, then discharged. Then, the API and the remaining excipients were mixed by incremental addition in a ratio of 1:2 (i.e., apixaban (or apixaban-containing material) to excipients). After each addition of excipients, mixing was performed at a speed of 10 rpm for 5 minutes. After the final addition of excipients, mixing was performed at a speed of 10 rpm for 15 minutes.

Hot-melt granulation was carried out using a twin-screw extruder. The screw assembly adopted a standard configuration, with a granulation temperature of 70-80°C, a screw speed of 300 rpm, and a feed speed of 40 rpm. The obtained granules were passed through a 20-mesh sieve to obtain apixaban granules.

**Table 17. Specific component information of Example 17**

| Component | mg per unit | wt% |
|---|---|---|
| Apixaban | 2.5 | 2.5 |
| Lactose monohydrate | 64.5 | 64.5 |
| Poloxamer 407 | 18.0 | 18.0 |
| Croscarmellose sodium | 15.0 | 15.0 |
| Total | 100.0 | 100.0 |

### Example 18

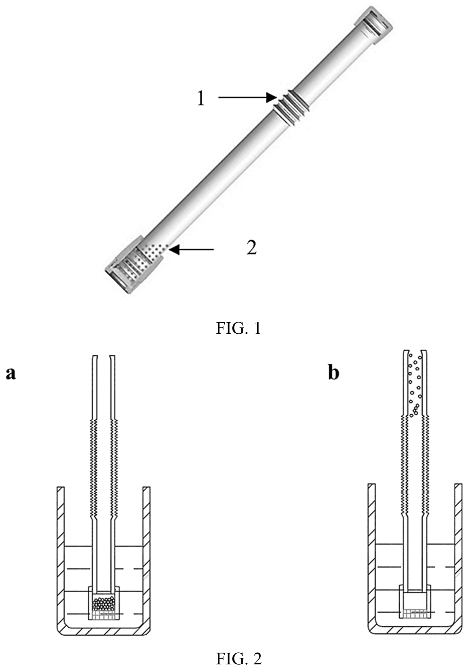

The carbidopa-levodopa granules obtained in Example 1 were filled into the AcuSiS^{®} as shown in FIG. 1 by the following process to obtain a carbidopa-levodopa granule formulation:
A polypropylene melt-blown nonwoven fabric disc was loaded into a high-density polyethylene pharmaceutical AcuSiS^{®} filter housing cap bottom piece, and then the high-density polyethylene pharmaceutical AcuSiS^{®} filter housing was snapped together with the bottom piece to form an AcuSiS^{®} filter; a polypropylene pharmaceutical AcuSiS^{®} straw was assembled with the AcuSiS^{®} filter to form an AcuSiS^{®} body; the granules after hot-melt granulation were filled into the AcuSiS^{®} body and sealed with a high-density polyethylene pharmaceutical AcuSiS^{®} cap.

The filled AcuSiS^{®} was sealed using a polyester/aluminum/polyethylene composite film bag, with a 0.25 g desiccant pouch added inside the bag.

As shown in FIG. 2, during use of the product of this example, no prior preparation is required. The polyester/aluminum/polyethylene composite film bag is directly opened, the cap is removed, and the product is placed in water, milk, or juice. Upon suction by the patient, the drug instantly disperses in the liquid and is subsequently swallowed.

### Example 19

The apixaban granules obtained in Example 16 were filled into AcuSiS^{®} according to the steps in Example 18 to obtain the apixaban formulation.

### Example 20

According to the formulation in Table 18, carbidopa-levodopa granules were prepared by the following hot-melt granulation process using the hot-melt adhesive Poloxamer 407:
Poloxamer 407 was pulverized and passed through a 40-mesh sieve to obtain Poloxamer 407 powder.

Carbidopa, levodopa, and other excipients were added to a square cone mixer and mixed at a speed of 10 rpm for 10 minutes, and the mixture was discharged.

Hot-melt granulation was carried out using a twin-screw extruder. The screw assembly adopted a standard configuration, with a granulation temperature of 70-90°C, a screw speed of 200 rpm, and a feed speed of 10 rpm. The obtained granules were passed through a 20-mesh sieve to obtain carbidopa-levodopa granules.

**Table 18. Specific component information of Example 20**

| Component | wt% |
|---|---|
| Carbidopa monohydrate | 7.5* |
| Levodopa | 27.6 |
| Mannitol | 33.4 |
| Poloxamer 407 | 18.5 |
| Low-substituted hydroxypropyl cellulose | 13.0 |
| Total | 100.0 |

| | |
|---|---|
| Note: * indicates the amount of carbidopa monohydrate, equivalent to 25 mg (6.9%) of carbidopa. | |

### Example 21

According to the formulation in Table 19, carbidopa-levodopa granules were prepared by the following hot-melt granulation process:
Poloxamer 407 was pulverized and passed through a 40-mesh sieve to obtain Poloxamer 407 powder.

Carbidopa, levodopa active pharmaceutical ingredients and excipients were added to a square cone mixer and mixed at a speed of 10 rpm for 10 minutes, and the mixture was discharged.

Hot-melt granulation was carried out using a twin-screw extruder. The screw assembly adopted a standard configuration, with a granulation temperature of 70-80°C, a screw speed of 400 rpm, and a feed speed of 15 rpm. The obtained granules were passed through a 20-mesh sieve to obtain carbidopa-levodopa granules.

**Table 19. Specific component information of Example 21**

| Component | mg per unit | wt% |
|---|---|---|
| Carbidopa monohydrate | 27.0* | 7.5 |
| Levodopa | 100.0 | 27.6 |
| Mannitol | 113.3 | 31.8 |
| Poloxamer 407 | 66.9 | 18.0 |
| Low-substituted | 54.6 | 15.1 |
| hydroxypropyl cellulose | | |
| Total | 361.8 | 100.0 |

| | | |
|---|---|---|
| Note: * indicates the amount of carbidopa monohydrate, equivalent to 25 mg (6.9%) of carbidopa. | | |

### Example 22

The carbidopa-levodopa granules obtained in Example 21 were filled into the AcuSiS^{®} as shown in FIG. 1 through the following process to obtain the carbidopa-levodopa granule formulation:
A polypropylene melt-blown nonwoven fabric disc was loaded into a high-density polyethylene pharmaceutical AcuSiS^{®} filter housing cap bottom piece, and then the high-density polyethylene pharmaceutical AcuSiS^{®} filter housing was snapped together with the bottom piece to form an AcuSiS^{®} filter; a polypropylene pharmaceutical AcuSiS^{®} straw was assembled with the AcuSiS^{®} filter to form an AcuSiS^{®} body; the granules after hot-melt granulation were filled into the AcuSiS^{®} body and sealed with a high-density polyethylene pharmaceutical AcuSiS^{®} cap.

The filled AcuSiS^{®} was sealed using a polyester/aluminum/polyethylene composite film bag.

### Example 23

The carbidopa-levodopa granule formulation was obtained according to Example 22, and the filled AcuSiS^{®} was sealed using a polyester/aluminum/polyethylene composite film bag, with a 0.25 g desiccant pouch added inside the bag.

### Example 24

According to the formulation in Table 20, carbidopa-levodopa granules were prepared by the hot-melt granulation process of Example 21.

**Table 20. Specific component information of Example 24**

| Component | mg per unit | wt% |
|---|---|---|
| Carbidopa monohydrate | 27.0* | 7.5 |
| Levodopa | 100.0 | 27.6 |
| Mannitol | 113.3 | 31.8 |
| Polyethylene glycol 6000 | 66.9 | 18.0 |
| Low-substituted hydroxypropyl cellulose | 54.6 | 15.1 |
| Total | 361.8 | 100.0 |

| | | |
|---|---|---|
| Note: * indicates the amount of carbidopa monohydrate, equivalent to 25 mg (6.9%) of carbidopa. | | |

### Example 25

According to the formulation in Table 21, the components except sodium stearyl fumarate were prepared into granules according to the hot-melt granulation process of Example 21.

The granules and sodium stearyl fumarate were added to a square cone mixer and mixed at a speed of 10 rpm for 5 minutes, and the mixture was discharged to obtain carbidopa-levodopa granules.

**Table 21. Specific component information of Example 25**

| Component | mg per unit | wt% |
|---|---|---|
| Carbidopa monohydrate | 27.0* | 7.3 |
| Levodopa | 100.0 | 27.1 |
| Mannitol | 113.3 | 30.7 |
| Poloxamer 407 | 66.9 | 18.1 |
| Low-substituted hydroxypropyl cellulose | 54.6 | 14.8 |
| Sodium stearyl fumarate | 7.2 | 2.0 |
| Total | 369.0 | 100.0 |

| | | |
|---|---|---|
| Note: * indicates the amount of carbidopa monohydrate, equivalent to 25 mg (6.9%) of carbidopa. | | |

### Example 26

According to the formulation in Table 22, voriconazole granules were prepared by the following hot-melt granulation process:
Poloxamer 407 was pulverized and passed through a 40-mesh sieve to obtain Poloxamer 407 powder. Voriconazole and other excipients were added to a square cone mixer and mixed at a speed of 10 rpm for 10 minutes, and the mixture was discharged. The resulting mixture was placed in a high-shear granulator with a heating jacket for granulation at a temperature of 70-80°C until uniform granules were formed. The hot-melt granules were passed through a 16-mesh sieve to obtain voriconazole granules.

**Table 22. Specific component information of Example 26**

| Component | mg per unit | wt% |
|---|---|---|
| Voriconazole | 50.0 | 41.7 |
| Mannitol | 25.9 | 21.6 |
| Poloxamer 407 | 25.9 | 21.6 |
| Low-substituted hydroxypropyl cellulose | 18.1 | 15.1 |
| Total | 119.9 | 100.0 |

### Example 27

According to the formulation in Table 23, voriconazole granules were prepared by the following hot-melt granulation process:
Poloxamer 407 was pulverized and passed through an 80-mesh sieve to obtain Poloxamer 407 powder. Voriconazole and other excipients were added to a square cone mixer and mixed at a speed of 10 rpm for 30 minutes, and the mixture was discharged. Hot-melt granulation was performed using a twin-screw extruder, with a granulation temperature of 70-80°C, a screw speed of 200 rpm, and a feed rate of 30%. The hot-melt granules were passed through a 16-mesh sieve to obtain voriconazole granules.

**Table 23. Specific component information of Example 27**

| Component | mg per unit | wt% |
|---|---|---|
| Voriconazole | 50.0 | 8.3 |
| Mannitol | 301.2 | 50.0 |
| Poloxamer 407 | 130.7 | 21.7 |
| Low-substituted hydroxypropyl cellulose | 120.5 | 20.0 |
| Total | 602.4 | 100.0 |

### Example 28

According to the formulation in Table 24, voriconazole granules were prepared by the hot-melt granulation process of Example 27.

**Table 24. Specific component information of Example 28**

| Component | mg per unit | wt% |
|---|---|---|
| Voriconazole | 200.0 | 41.7 |
| Mannitol | 107.0 | 22.3 |
| Poloxamer 407 | 86.3 | 18.0 |
| Low-substituted hydroxypropyl cellulose | 86.3 | 18.0 |
| Total | 479.6 | 100.0 |

### Example 29

According to the formulation in Table 25, the granules from Example 28 were added with 0.5% magnesium stearate and 0.5% strawberry flavor, then placed in a square cone mixer, and mixed at a speed of 10 rpm for 5 minutes to obtain voriconazole granules.

**Table 25. Specific component information of Example 29**

| Component | mg per unit | wt% |
|---|---|---|
| Voriconazole | 200.0 | 41.3 |
| Mannitol | 107.0 | 22.1 |
| Poloxamer 407 | 86.3 | 17.8 |
| Low-substituted hydroxypropyl cellulose | 86.3 | 17.8 |
| Magnesium stearate | 2.4 | 0.5 |
| Strawberry flavor | 2.4 | 0.5 |
| Total | 484.4 | 100.0 |

### Example 30

According to the formulation in Table 26, voriconazole granules were prepared by the hot-melt granulation process of Example 26.

**Table 26. Specific component information of Example 30**

| Component | mg per unit | wt% |
|---|---|---|
| Voriconazole | 50.0 | 63.0 |
| Poloxamer 407 | 14.3 | 18.0 |
| Low-substituted hydroxypropyl cellulose | 14.3 | 18.0 |
| Colloidal silica | 0.8 | 1.0 |
| Total | 79.4 | 100.0 |

### Example 31

According to the formulation in Table 27, voriconazole granules were prepared by the following hot-melt granulation process:
Poloxamer 407 was pulverized and passed through a 40-mesh sieve to obtain Poloxamer 407 powder. Voriconazole and other excipients were added to a square cone mixer and mixed at a speed of 10 rpm for 10 minutes, and the mixture was discharged. The resulting mixture was placed in a high-shear granulator with a heating jacket for granulation at a temperature of 80-90°C until uniform granules were formed. The hot-melt granules were passed through a 14-mesh sieve to obtain voriconazole granules.

**Table 27. Specific component information of Example 31**

| Component | mg per unit | wt% |
|---|---|---|
| Voriconazole | 300.0 | 54.5 |
| Mannitol | 102.9 | 18.7 |
| Poloxamer 407 | 66.1 | 12.0 |
| Low-substituted hydroxypropyl cellulose | 76.0 | 13.8 |
| Colloidal silica | 5.5 | 1.0 |
| Total | 550.5 | 100.0 |

### Example 32

According to the formulation in Table 28, voriconazole granules were prepared by the following hot-melt granulation process:
Poloxamer 188 was pulverized and passed through an 80-mesh sieve to obtain Poloxamer 188 powder. Voriconazole and other excipients were added to a square cone mixer and mixed at a speed of 10 rpm for 30 minutes, and the mixture was discharged. Hot-melt granulation was performed using a twin-screw extruder, with a granulation temperature of 60-70°C, a screw speed of 400 rpm, and a feed rate of 40%. The hot-melt granules were passed through a 16-mesh sieve to obtain voriconazole granules.

**Table 28. Specific component information of Example 32**

| Component | mg per unit | wt% |
|---|---|---|
| Voriconazole | 300.0 | 54.5 |
| Mannitol | 137.7 | 25.0 |
| Poloxamer 188 | 107.3 | 19.5 |
| Colloidal silica | 5.5 | 1.0 |
| Total | 550.5 | 100.0 |

### Example 33

According to the formulation in Table 29, Poloxamer 407 was pulverized and passed through an 80-mesh sieve to obtain Poloxamer 407 powder. Voriconazole granules were prepared by the hot-melt granulation process of Example 32.

**Table 29. Specific component information of Example 33**

| Component | mg per unit | wt% |
|---|---|---|
| Voriconazole | 50.0 | 51.6 |
| Mannitol | 18.3 | 18.9 |
| Poloxamer 407 | 17.9 | 18.5 |
| Microcrystalline cellulose | 9.7 | 10.0 |
| Colloidal silica | 1.0 | 1.0 |
| Total | 96.9 | 100.0 |

### Example 34

According to the formulation in Table 30, Poloxamer 407 was pulverized and passed through an 80-mesh sieve to obtain Poloxamer 407 powder. Voriconazole granules were prepared by the hot-melt granulation process of Example 32.

**Table 30. Specific component information of Example 34**

| Component | mg per unit | wt% |
|---|---|---|
| Voriconazole | 50.0 | 51.6 |
| Mannitol | 18.3 | 18.9 |
| Poloxamer 407 | 17.9 | 18.5 |
| Pregelatinized starch | 9.7 | 10.0 |
| Colloidal silica | 1.0 | 1.0 |
| Total | 96.9 | 100.0 |

### Example 35

According to the formulation in Table 31, voriconazole granules were prepared by the following hot-melt granulation process:
Poloxamer 407 was pulverized and passed through an 80-mesh sieve to obtain Poloxamer 407 powder. Voriconazole and other excipients were added to a square cone mixer and mixed at a speed of 10 rpm for 30 minutes, and the mixture was discharged. Hot-melt granulation was performed using a twin-screw extruder, with a granulation temperature of 70-80°C, a screw speed of 400 rpm, and a feed rate of 25%. The hot-melt granules were passed through a 16-mesh sieve to obtain voriconazole granules.

**Table 31. Specific component information of Example 35**

| Component | mg per unit | wt% |
|---|---|---|
| Voriconazole | 300.0 | 54.5 |
| Mannitol | 82.6 | 15.0 |
| Poloxamer 407 | 107.3 | 19.5 |
| Low-substituted hydroxypropyl cellulose | 55.1 | 10.0 |
| Colloidal silica | 5.5 | 1.0 |
| Total | 550.5 | 100.0 |

### Example 36

According to the formulation in Table 32, voriconazole granules were prepared by the hot-melt granulation process of Example 35.

**Table 32. Specific component information of Example 36**

| Component | mg per unit | wt% |
|---|---|---|
| Voriconazole | 300.0 | 54.5 |
| Mannitol | 94.7 | 17.2 |
| Poloxamer 407 | 82.6 | 15.0 |
| Low-substituted hydroxypropyl cellulose | 67.7 | 12.3 |
| Colloidal silica | 5.5 | 1.0 |
| Total | 550.5 | 100.0 |

### Example 37

According to the formulation in Table 33, the granules from Example 35 were added with 0.5% colloidal silica, 0.3% aspartame, and 0.7% orange flavor, then placed in a square cone mixer, and mixed at a speed of 10 rpm for 5 minutes to obtain voriconazole granules.

**Table 33. Specific component information of Example 37**

| Component | mg per unit | wt% |
|---|---|---|
| Voriconazole | 300.0 | 53.7 |
| Mannitol | 82.6 | 14.8 |
| Poloxamer 407 | 107.3 | 19.2 |
| Low-substituted hydroxypropyl cellulose | 55.1 | 9.8 |
| Colloidal silica | 5.5 | 1.0 |
| Colloidal silica | 2.8 | 0.5 |
| Aspartame | 1.7 | 0.3 |
| Orange flavor | 3.9 | 0.7 |
| Total | 558.9 | 100.0 |

### Example 38

According to the formulation in Table 34, voriconazole granules were prepared by the following hot-melt granulation process:
Poloxamer 407 was pulverized and passed through an 80-mesh sieve to obtain Poloxamer 407 powder. Voriconazole and other excipients were added to a square cone mixer and mixed at a speed of 10 rpm for 30 minutes, and the mixture was discharged. Hot-melt granulation was performed using a twin-screw extruder, with a granulation temperature of 60-70°C, a screw speed of 400 rpm, and a feed rate of 30%. The hot-melt granules were passed through a 16-mesh sieve to obtain voriconazole granules.

**Table 34. Specific component information of Example 38**

| Component | mg per unit | wt% |
|---|---|---|
| Voriconazole | 300.0 | 33.0 |
| Mannitol | 255.5 | 28.1 |
| Poloxamer 407 | 162.7 | 17.9 |
| Low-substituted hydroxypropyl cellulose | 181.8 | 20.0 |
| Colloidal silica | 9.1 | 1.0 |
| Total | 909.1 | 100.0 |

### Example 39

According to the formulation in Table 35, the granules from Example 38 were added with 0.5% aspartame and 0.5% orange flavor, then placed in a square cone mixer, and mixed at a speed of 10 rpm for 5 minutes to obtain voriconazole granules.

**Table 35. Specific component information of Example 39**

| Component | mg per unit | wt% |
|---|---|---|
| Voriconazole | 300.0 | 32.7 |
| Mannitol | 255.5 | 27.8 |
| Poloxamer 407 | 162.7 | 17.7 |
| Low-substituted hydroxypropyl cellulose | 181.8 | 19.8 |
| Colloidal silica | 9.1 | 1.0 |
| Aspartame | 4.6 | 0.5 |
| Orange flavor | 4.6 | 0.5 |
| Total | 918.3 | 100.0 |

### Example 40

According to the formulation in Table 36, voriconazole granules were prepared by the following hot-melt granulation process:
Poloxamer 407 was pulverized and passed through an 80-mesh sieve to obtain Poloxamer 407 powder. Voriconazole and other excipients were added to a square cone mixer and mixed at a speed of 10 rpm for 20 minutes, and the mixture was discharged. Hot-melt granulation was performed using a twin-screw extruder, with a granulation temperature of 60-70°C, a screw speed of 800 rpm, and a feed rate of 80%. The hot-melt granules were passed through a 16-mesh sieve to obtain voriconazole granules.

**Table 36. Specific component information of Example 40**

| Component | mg per unit | wt% |
|---|---|---|
| Voriconazole | 300.0 | 43.9 |
| Mannitol | 192.0 | 28.1 |
| Poloxamer 407 | 95.7 | 14.0 |
| Low-substituted hydroxypropyl cellulose | 88.8 | 13.0 |
| Colloidal silica | 6.8 | 1.0 |
| Total | 683.3 | 100.0 |

### Example 41

According to the formulation in Table 37, voriconazole granules were prepared by the following hot-melt granulation process:
Poloxamer 407 was pulverized and passed through a 40-mesh sieve to obtain Poloxamer 407 powder. Voriconazole and other excipients were added to a square cone mixer and mixed at a speed of 10 rpm for 20 minutes, and the mixture was discharged. Hot-melt granulation was performed using a twin-screw extruder, with a granulation temperature of 60-70°C, a screw speed of 100 rpm, and a feed rate of 10%. The hot-melt granules were passed through a 16-mesh sieve to obtain voriconazole granules.

**Table 37. Specific component information of Example 41**

| Component | mg per unit | wt% |
|---|---|---|
| Voriconazole | 300.0 | 33.0 |
| Mannitol | 331.8 | 36.5 |
| Poloxamer 407 | 227.3 | 25.0 |
| Low-substituted hydroxypropyl cellulose | 45.5 | 5.0 |
| Colloidal silica | 4.5 | 0.5 |
| Total | 909.1 | 100.0 |

### Example 42

According to the formulation in Table 38, voriconazole granules were prepared by the following hot-melt granulation process:
Poloxamer 407 was pulverized and passed through an 80-mesh sieve to obtain Poloxamer 407 powder. Voriconazole, mannitol, Poloxamer 407 powder, low-substituted hydroxypropyl cellulose, and colloidal silica were added to a square cone mixer and mixed at a speed of 10 rpm for 30 minutes, and the mixture was discharged. Hot-melt granulation was performed using a twin-screw extruder, with a granulation temperature of 70-80°C, a screw speed of 200 rpm, and a feed rate of 20%. The hot-melt granules were passed through a 16-mesh sieve, and the sieved granules were mixed with the remaining excipients in a square cone mixer at a speed of 10 rpm for 5 minutes to obtain voriconazole granules.

**Table 38. Specific component information of Example 42**

| Component | mg per unit | wt% |
|---|---|---|
| Voriconazole | 300.0 | 39.0 |
| Mannitol | 153.8 | 20.0 |
| Poloxamer 407 | 165.4 | 21.5 |
| Low-substituted hydroxypropyl cellulose | 133.1 | 17.3 |
| Colloidal silica | 3.8 | 0.5 |
| Magnesium stearate | 3.8 | 0.5 |
| Aspartame | 4.6 | 0.6 |
| Orange flavor | 4.6 | 0.6 |
| Total | 769.1 | 100.0 |

### Example 43

As shown in FIG. 1, the voriconazole granules obtained in Example 28 were filled into an AcuSiS^{®} oral administration delivery formulation kit containing voriconazole by the following process:
A polypropylene melt-blown nonwoven fabric disc was loaded into a high-density polyethylene pharmaceutical AcuSiS^{®} filter housing bottom piece, and then the high-density polyethylene pharmaceutical AcuSiS^{®} filter housing was snapped together with the bottom piece to form an AcuSiS^{®} filter; a polypropylene pharmaceutical AcuSiS^{®} straw was assembled with the AcuSiS^{®} filter to form an AcuSiS^{®} body; the voriconazole granules obtained in Example 28 were filled into the AcuSiS^{®} body and sealed with a high-density polyethylene pharmaceutical AcuSiS^{®} cap.

### Example 44

The voriconazole granules obtained in Example 29 were filled into an AcuSiS^{®} oral administration delivery formulation kit containing voriconazole according to the steps in Example 43. The filled AcuSiS^{®} was sealed using a polyester/aluminum/polyethylene composite film bag, with a 0.25 g desiccant pouch added inside the composite film bag.

As shown in FIG. 2, during use of the product of this example, no prior preparation is required. The polyester/aluminum/polyethylene composite film bag is directly opened, the AcuSiS^{®} is taken out, the cap is removed, and the product is placed in water, milk, or juice. Upon suction by the patient, the drug instantly disperses in the liquid and is subsequently swallowed.

### Example 45

According to the formulation in Table 39, voriconazole granules were prepared by the hot-melt granulation process in Example 35 using the hot-melt adhesive polyethylene glycol 6000.

**Table 39. Specific component information of Example 45**

| Component | wt% |
|---|---|
| Voriconazole | 54.5 |
| Mannitol | 15.0 |
| Polyethylene glycol 6000 | 19.5 |
| Low-substituted hydroxypropyl cellulose | 10.0 |
| Colloidal silica | 1.0 |
| Total | 100.0 |

### Example 46

According to the formulation in Table 40, voriconazole granules were prepared by the hot-melt granulation process of Example 35.

**Table 40. Specific component information of Example 46**

| Component | wt% |
|---|---|
| Voriconazole | 54.5 |
| Mannitol | 15.0 |
| Polyethylene glycol 6000 | 19.2 |
| Glyceryl monostearate | 0.3 |
| Low-substituted hydroxypropyl cellulose | 10.0 |
| Colloidal silica | 1.0 |
| Total | 100.0 |

### Example 47

According to the formulation in Table 41, voriconazole granules were prepared by the hot-melt granulation process of Example 35.

**Table 41. Specific component information of Example 47**

| Component | wt% |
|---|---|
| Voriconazole | 54.5 |
| Mannitol | 10.2 |
| Polyethylene glycol 6000 | 21.6 |
| Xanthan gum | 2.7 |
| Low-substituted hydroxypropyl cellulose | 10.0 |
| Colloidal silica | 1.0 |
| Total | 100.0 |

### Example 48

According to the formulation in Table 42, voriconazole granules were prepared by the hot-melt granulation process of Example 35.

**Table 42. Specific component information of Example 48**

| Component | wt% |
|---|---|
| Voriconazole | 54.5 |
| Polyethylene glycol 6000 | 19.5 |
| Xanthan gum | 15.0 |
| Low-substituted hydroxypropyl cellulose | 10.0 |
| Colloidal silica | 1.0 |
| Total | 100.0 |

### Example 49

According to the formulation in Table 43, voriconazole granules were prepared by the hot-melt granulation process of Example 35.

**Table 43. Specific component information of Example 49**

| Component | wt% |
|---|---|
| Voriconazole | 54.5 |
| Mannitol | 10.0 |
| Polyethylene glycol 6000 | 18.0 |
| Soy lecithin | 1.0 |
| Croscarmellose sodium | 15.5 |
| Colloidal silica | 1.0 |
| Total | 100.0 |

### Example 50

According to the formulation in Table 44, voriconazole granules were prepared by the hot-melt granulation process of Example 35.

**Table 44. Specific component information of Example 50**

| Component | wt% |
|---|---|
| Voriconazole | 54.5 |
| Mannitol | 9.6 |
| Polyethylene glycol 6000 | 19.5 |
| Xanthan gum | 5.4 |
| Croscarmellose sodium | 10.0 |
| Colloidal silica | 1.0 |
| Total | 100.0 |

### Example 51

According to the formulation in Table 45, voriconazole granules were prepared by the hot-melt granulation process of Example 35.

**Table 45. Specific component information of Example 51**

| Component | wt% |
|---|---|
| Voriconazole | 54.5 |
| Sorbitol | 10.2 |
| Polyethylene glycol 6000 | 18.0 |
| Xanthan gum | 5.4 |
| Croscarmellose sodium | 10.9 |
| Colloidal silica | 1.0 |
| Total | 100.0 |

### Example 52

According to the formulation in Table 46, voriconazole granules were prepared by the hot-melt granulation process in Example 35 using the hot-melt adhesives polyethylene glycol 6000 and Poloxamer 407.

**Table 46. Specific component information of Example 52**

| Component | wt% |
|---|---|
| Voriconazole | 54.5 |
| Mannitol | 15.0 |
| Polyethylene glycol 6000 | 16.5 |
| Poloxamer 407 | 3.0 |
| Croscarmellose sodium | 10.0 |
| Colloidal silica | 1.0 |
| Total | 100.0 |

### Comparative Examples 1 to 3

According to the formulation in Table 47, granules were prepared by the hot-melt granulation process in Example 20 using the hot-melt adhesives glyceryl behenate 888 ATO and polyethylene glycol-32 stearate.

**Table 47. Specific component information of Example 20 and Comparative Examples 1 to 3**

| Component | Example 20 wt% | Comparative Example 1 wt% | Comparative Example 2 wt% | Comparative Example 3 wt% |
|---|---|---|---|---|
| Carbidopa monohydrate | 7.5* | 7.5* | 7.5* | 7.5* |
| Levodopa | 27.6 | 27.6 | 27.6 | 27.6 |
| Mannitol | 33.4 | 20.9 | 33.4 | 33.4 |
| Poloxamer 407 | 18.5 | 31.0 | N/A | N/A |
| Glyceryl behenate 888 ATO | N/A | N/A | 18.5 | N/A |
| Polyethylene glycol-32 stearate | N/A | N/A | N/A | 18.5 |
| Low-substituted hydroxypropyl cellulose | 13.0 | 13.0 | 13.0 | 13.0 |
| Total | 100.0 | 100.0 | 100.0 | 100.0 |

| | | | | |
|---|---|---|---|---|
| Note: * indicates the amount of carbidopa monohydrate, equivalent to 25 mg (6.9%) of carbidopa. | | | | |

### Comparative Example 4

According to the formulation in Table 48, voriconazole granules were prepared by the hot-melt granulation process in Example 35 using the hot-melt adhesive glyceryl behenate 888 ATO.

**Table 48. Specific component information of Comparative Example 4**

| Component | wt% |
|---|---|
| Voriconazole | 54.5 |
| Mannitol | 15.0 |
| Glyceryl behenate 888 ATO | 19.5 |
| Low-substituted hydroxypropyl cellulose | 10.0 |
| Colloidal silica | 1.0 |
| Total | 100.0 |

### Effect Example 1 Dissolution Test Detection

For the carbidopa-levodopa granules of Examples 1-3 and Examples 24-25, the dissolution method used was USP Method 1 (basket method) at a rotation speed of 50 rpm. The dissolution medium was 0.1N hydrochloric acid at pH 1.0, with a volume of 750 mL. Samples were taken at 5, 10, 15, 20, and 30 minutes for testing. The dissolution results for Examples 1-3 and Examples 24-25 are shown in Tables 48-50 and Tables 55-56, respectively. The release of carbidopa and levodopa was rapid, with dissolution exceeding 90% at 5 minutes. For the apixaban granules of Examples 16 and 17, the dissolution method used was USP Method 2 (paddle method) at a rotation speed of 75 rpm. The dissolution medium was 0.05 M sodium phosphate buffer containing 0.05% SLS at pH 6.8, with a volume of 900 mL. Samples were taken at 5, 10, 20, 30, and 45 minutes for testing. The dissolution results for Examples 16 and 17 are shown in Table 52, with the dissolution of apixaban exceeding 85% at 5 minutes.

For the carbidopa-levodopa granules of Example 20 and Comparative Examples 2-3, the dissolution method used was USP Method 1 (basket method) at a rotation speed of 50 rpm. The dissolution medium was 0.1N hydrochloric acid at pH 1.0, with a volume of 750 mL. Samples were taken at 5, 10, 15, 20, and 30 minutes for testing. The dissolution results are shown in Tables 53 and 54. In Example 20, the release of carbidopa and levodopa was rapid, with dissolution exceeding 90% at 5 minutes. In contrast, the release of levodopa and carbidopa in Comparative Example 2 and Comparative Example 3 was slow, with dissolution at 5 minutes of 23.4% and 24.5% (Comparative Example 2), and 70.0% and 68.4% (Comparative Example 3), respectively.

**Table 49. Dissolution test results of the carbidopa-levodopa granules obtained in Example 1**

| Sampling point/min | Carbidopa dissolution% | Levodopa dissolution% |
|---|---|---|
| 5 | 97.5 | 99.8 |
| 10 | 97.7 | 100.1 |
| 15 | 97.7 | 100.2 |
| 20 | 97.5 | 100.1 |
| 30 | 97.7 | 100.3 |

**Table 50. Dissolution test results of the carbidopa-levodopa granules obtained in Example 2**

| Sampling point/min | Carbidopa dissolution% | Levodopa dissolution% |
|---|---|---|
| 5 | 99.1 | 98.8 |
| 10 | 100.0 | 99.9 |
| 15 | 99.8 | 99.9 |
| 20 | 100.0 | 100.0 |
| 30 | 100.0 | 100.0 |

**Table 51. Dissolution test results of the carbidopa-levodopa granules obtained in Example 3**

| Sampling point/min | Carbidopa dissolution% | Levodopa dissolution% |
|---|---|---|
| 5 | 98.2 | 100.8 |
| 10 | 98.6 | 101.5 |
| 15 | 98.3 | 101.3 |
| 20 | 98.4 | 101.6 |
| 30 | 98.7 | 101.6 |

**Table 52. Dissolution test results of apixaban granules obtained in Example 16 and Example 17**

| Sampling point/min | Example 16 Apixaban dissolution% | Example 17 Apixaban dissolution% |
|---|---|---|
| 5 | 92.1 | 86.6 |
| 10 | 96.5 | 93.2 |
| 20 | 96.3 | 95.9 |
| 30 | 96.1 | 96.1 |
| 45 | 96.9 | 96.3 |

**Table 53. Levodopa dissolution of the carbidopa-levodopa granules obtained in Example 20 and Comparative Examples 2-3**

| Sampling point/min | Example 20 Dissolution% | Comparative Example 2 Dissolution% | Comparative Example 3 Dissolution% |
|---|---|---|---|
| 5 | 95.3% | 23.4% | 70.0% |
| 10 | 95.6% | 29.4% | 87.5% |
| 15 | 95.5% | 32.4% | 91.8% |
| 20 | 95.7% | 34.7% | 92.4% |
| 30 | 95.6% | 38.4% | 93.0% |

**Table 54. Carbidopa dissolution of the carbidopa-levodopa granules obtained in Example 20 and Comparative Examples 2-3**

| Sampling point/min | Example 20 Dissolution% | Comparative Example 2 Dissolution% | Comparative Example 3 Dissolution% |
|---|---|---|---|
| 5 | 94.8% | 24.5% | 68.4% |
| 10 | 94.5% | 30.3% | 85.0% |
| 15 | 94.2% | 33.3% | 89.0% |
| 20 | 93.9% | 35.4% | 89.1% |
| 30 | 93.1% | 38.9% | 89.3% |

**Table 55. Dissolution test results of the carbidopa-levodopa granules obtained in Example 24**

| Sampling point/min | Carbidopa dissolution% | Levodopa dissolution% |
|---|---|---|
| 5 | 83.7 | 83.2 |
| 10 | 96.6 | 96.7 |
| 15 | 96.5 | 96.5 |
| 20 | 96.6 | 96.6 |
| 30 | 96.5 | 96.5 |

**Table 56. Dissolution test results of the carbidopa-levodopa granules obtained in Example 25**

| Sampling point/min | Carbidopa dissolution% | Levodopa dissolution% |
|---|---|---|
| 5 | 96.3 | 95.4 |
| 10 | 98.3 | 97.4 |
| 15 | 99.1 | 98.2 |
| 20 | 99.5 | 98.7 |
| 30 | 100.4 | 99.3 |

### Effect Example 2 Raw Material Compatibility and Formulation Stability Testing of Carbidopa-Levodopa Granules

For the formulation of carbidopa-levodopa granules, compatibility studies of the raw materials and excipients under conditions of 40°C/75% RH revealed, as shown in Tables 57 and 58, that among all excipients, Poloxamer 407 exhibited poor compatibility with both carbidopa and levodopa active pharmaceutical ingredients. After preparation into the formulation, there was a risk of exceeding the limit for related substances in terms of stability.

The carbidopa-levodopa granule formulations obtained from Example 18 and Examples 22-23 were placed under conditions of 40°C/75% RH and sampled for testing at 1 month and 6 months, respectively, with the test indicators being dissolution, related substances, and hydrazine content. The results are shown in Tables 59-61. After 6 months of accelerated testing, the dissolution of the carbidopa-levodopa granules did not show significant changes, the related substances were far below the limits, and the hydrazine content was extremely low, far below the limit of 50 ppm. This indicates that the use of hot-melt granulation process and moisture-proof packaging can significantly improve the compatibility of carbidopa and levodopa with Poloxamer 407 and increase the stability of the formulation.

**Table 57. Results of compatibility study on carbidopa-levodopa granule raw materials 1**

| Sample (40°C / 75%RH) | Total impurities (%) | | | Appearance |
|---|---|---|---|---|
| | 0 Days | 14 Days | 28 Days | |
| Carbidopa | 0.10 | 0.08 | 0.07 | No change |
| Carbidopa: Mannitol (1:10) | 0.09 | 0.14 | 0.18 | No change |
| Carbidopa: Poloxamer 407 (1:10) | 0.08 | 2.92 | 4.27 | Changed from a white powder to a pale yellow mass |
| Carbidopa: Low-substituted hydroxypropyl cellulose (1:5) | 0.12 | 0.08 | 0.12 | No change |

**Table 58. Results of compatibility study on carbidopa-levodopa granule raw materials 2**

| Sample (40°C / 75%RH) | Total impurities (%) | | | Appearance |
|---|---|---|---|---|
| | 0 Days | 14 Days | 28 Days | |
| Levodopa | Not detected | 0.03 | 0.07 | No change |
| Levodopa: Mannitol (1:10) | Not detected | 0.10 | 0.14 | No change |
| Levodopa: Poloxamer 407 (1:10) | Not detected | 2.85 | 4.21 | Changed from a white powder to a dark brown mass |
| Carbidopa: Low- | Not | 0.07 | 0.04 | No change |
| substituted hydroxypropyl cellulose (1:5) | detected | | | |

**Table 59. Stability test results of the carbidopa-levodopa granule formulation of Example 18**

| Test Item | Acceptance criteria | | | 0 Months | | 1 Month | | 6 Months | |
|---|---|---|---|---|---|---|---|---|---|
| | Time/min | % Release | | % Release | | | | | |
| | | Carbidopa (CD) | Levodopa (LD) | CD | LD | CD | LD | CD | LD |
| Dissolution | 5 | > 85 | > 85 | 97.9 | 97.7 | 91.3 | 94.0 | 98.0 | 99.0 |
| | 10 | N/A | N/A | 98.6 | 98.2 | 94.4 | 97.1 | 99.2 | 100.4 |
| | 15 | N/A | N/A | 98.6 | 98.4 | 94.6 | 97.4 | 99.0 | 100.0 |
| | 20 | N/A | N/A | 98.7 | 98.5 | 94.7 | 97.4 | 98.9 | 99.9 |
| | 30 | N/A | N/A | 98.8 | 98.5 | 94.7 | 97.5 | 99.0 | 100.1 |
| Related substances | CD | DHPA% | ≤ 1.0% | 0.04 | | 0.07 | | 0.11 | |
| | | Methyldopa% | ≤ 0.5% | 0.10 | | 0.13 | | 0.12 | |
| | | Maximum single unknown impurity% | ≤ 0.2% | Not detected | | Not detected | | Not detected | |
| | | Total impurities% | ≤ 2.0% | 0.14 | | 0.19 | | 0.22 | |
| | LD | Maximum single unknown impurity% | ≤ 0.1% | 0.02 | | Not detected | | Not detected | |
| | | Total impurities% | ≤ 0.3% | 0.02 | | Not detected | | Not detected | |
| Hydrazine | ≤ 50 ppm | | | 2.8 | | 3.6 | | 5.0 | |

**Table 60. Stability test results of the carbidopa-levodopa granule formulation of Example 22**

| Test Item | Acceptance criteria | | | 0 Months | | 1 Month | | 6 Months | |
|---|---|---|---|---|---|---|---|---|---|
| Dissolution | Time/min | % Release | | % Release | | | | | |
| | | Carbidopa (CD) | Levodopa (LD) | CD | LD | CD | LD | CD | LD |
| | 5 | > 85 | > 85 | 94.4 | 94.9 | 94.2 | 95.2 | 94.6 | 93.9 |
| | 10 | N/A | N/A | 94.2 | 94.9 | 94.6 | 95.9 | 94.4 | 93.8 |
| | 15 | N/A | N/A | 94.2 | 94.8 | 94.5 | 95.9 | 94.1 | 93.8 |
| | 20 | N/A | N/A | 94.1 | 94.8 | 94.7 | 96.0 | 94.0 | 93.9 |
| | 30 | N/A | N/A | 94.0 | 94.8 | 94.9 | 96.3 | 94.2 | 93.9 |
| Related substances | CD | DHPA% | ≤ 1.0% | 0.11 | | 0.20 | | 0.45 | |
| | | Methyldopa% | ≤ 0.5% | 0.16 | | 0.19 | | 0.30 | |
| | | Maximum single unknown impurity% | ≤ 0.2% | Not detected | | Not detected | | Not detected | |
| | | Total impurities% | ≤ 2.0% | 0.28 | | 0.39 | | 0.75 | |
| | LD | Maximum single unknown impurity% | ≤ 0.1% | Not detected | | Not detected | | Not detected | |
| | | Total impurities% | ≤ 0.3% | Not detected | | Not detected | | Not detected | |
| Hydrazine | ≤ 50 ppm | | | 4.4 | | 6.6 | | 9.8 | |

**Table 61. Stability test results of the carbidopa-levodopa granule formulation of Example 23**

| Test Item | Acceptance criteria | | | 0 Months | | 1 Month | | 6 Months | |
|---|---|---|---|---|---|---|---|---|---|
| Dissolution | Time/min | % Release | | % Release | | | | | |
| | | Carbidopa (CD) | Levodopa (LD) | CD | LD | CD | LD | CD | LD |
| | 5 | > 85 | > 85 | 94.9 | 95.7 | 95.5 | 96.6 | 94.2 | 93.0 |
| | 10 | N/A | N/A | 94.9 | 95.8 | 95.7 | 96.9 | 93.6 | 92.5 |
| | 15 | N/A | N/A | 94.9 | 95.8 | 95.7 | 96.9 | 93.5 | 92.6 |
| | 20 | N/A | N/A | 94.9 | 95.8 | 95.5 | 96.8 | 93.5 | 92.6 |
| | 30 | N/A | N/A | 94.9 | 95.8 | 95.8 | 97.0 | 93.4 | 92.5 |
| Related substances | CD | DHPA% | ≤ 1.0% | 0.07 | | 0.17 | | 0.33 | |
| | | Methyldopa% | ≤ 0.5% | 0.14 | | 0.22 | | 0.26 | |
| | | Maximum single unknown impurity% | ≤ 0.2% | Not detected | | Not detected | | Not detected | |
| | | Total impurities% | ≤ 2.0% | 0.21 | | 0.39 | | 0.58 | |
| | LD | Maximum single unknown impurity% | ≤ 0.1% | Not detected | | Not detected | | Not detected | |
| | | Total impurities% | ≤ 0.3% | Not detected | | Not detected | | Not detected | |
| Hydrazine | ≤ 50 ppm | | | 4.7 | | 9.0 | | 6.0 | |

Hydrazine is a genotoxic substance, and its content in the formulation should be strictly controlled. In commercially available compound formulation products containing levodopa and carbidopa, the content of hydrazine is often high during product storage and use, posing significant safety risks to patients. Testing revealed that the hydrazine content in samples of the commercially available carbidopa/levodopa formulations Sinemet CR and Rytary was 29.6 ppm and 72.3 ppm, respectively; whereas the carbidopa-levodopa granules prepared using the Poloxamer 407 formulation and the hot-melt granulation process exhibited good stability and extremely low hydrazine content, not exceeding 10 ppm, with some excellent products having only 3-5 ppm, demonstrating significant advantages.

### Effect Example 3: Sizing process observations

The sizing process observations for the granules obtained in Example 20 and Comparative Examples 1-3 are shown in Table 62. When the amount of hot-melt adhesive was 31% (Comparative Example 1), the granules formed lumps after granulation, and after cooling, the texture was hard, making it difficult to size.

**Table 62. Process observations for Example 20 and Comparative Examples 1-3**

| | Process observations |
|---|---|
| Example 20 | The granules post-granulation were friable and easy to size |
| Comparative Example 1 | The granules post-granulation formed lumps (excessive adhesive) and, after cooling, became hard in texture, making it difficult to size |
| Comparative Example 2 | The granules post-granulation were friable and easy to size |
| Comparative Example 3 | The granules post-granulation were friable and easy to size |

### Effect Example 4 Bioequivalence study

Carbidopa-levodopa granules (25 mg/100 mg, same process and packaging as in Example 18, different batch) and carbidopa-levodopa tablets (25 mg/100 mg, IR, manufactured by Mylan, commercially available) were used to conduct a bioequivalence study in healthy adult subjects (N = 12) under fasting conditions.

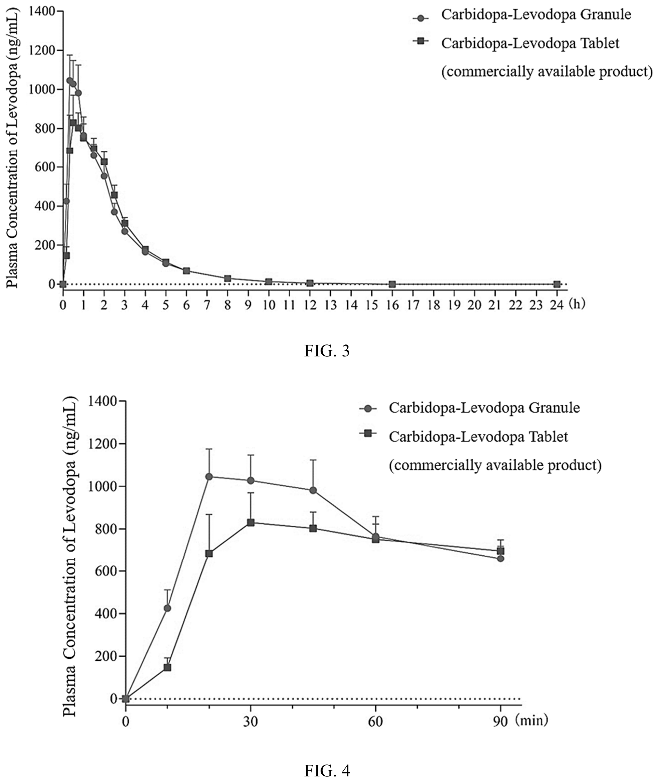

An open-label, parallel, randomized, two-treatment, two-period, two-sequence, single-dose, crossover Phase I clinical study showed that, expressed as mean, the time to peak (tₘₐₓ) of levodopa from carbidopa-levodopa granules and carbidopa-levodopa tablets in humans *in vivo* was 40.0 min and 57.7 min, respectively, with the former being 17.7 minutes faster than the latter; expressed as median, the time to peak (tₘₐₓ) of levodopa from carbidopa-levodopa granules and carbidopa-levodopa tablets in humans *in vivo* was 30.0 min and 52.5 min, respectively, with the former being 22.5 minutes faster than the latter (Table 63 and FIGS. 3-4); these results are consistent with the trend observed in *in vitro* release (Table 64).

**Table 63. Pharmacokinetic data of carbidopa-levodopa granules and carbidopa-levodopa tablets in healthy adult subjects under fasting conditions**

| **Pharmacokinetic data of levodopa (n = 12)** | | | | | | | | | | | | |
|---|---|---|---|---|---|---|---|---|---|---|---|---|
| | **Carbidopa-levodopa granules (25 mg/100 mg)** | | | | | | **Carbidopa-levodopa tablets (25 mg/100 mg)** | | | | | |
| | Mean | SD | CV (%) | Min | Median | Max | Mean | SD | CV (%) | Min | Median | Max |
| tₘₐₓ, min | 40.0 | 21.2 | 53. 1 | 20.0 | 30.0 | 90.0 | 57.7 | 33.4 | 57. 9 | 20.0 | 52.5 | 120.0 |
| Cₘₐₓ, ng/mL | 1376. 9 | 422. 7 | 30. 7 | 524.7 | 1356. 0 | 2244. 1 | 1146. 3 | 468. 3 | 40. 9 | 721.5 | 1020. 8 | 2396. 7 |
| AUC_{0-τ}, h·ng/m L | 2443. 5 | 537. 2 | 22. 0 | 1802. 4 | 2280. 3 | 3692. 8 | 2392. 0 | 418. 1 | 17. 5 | 1901. 6 | 2305. 9 | 3068. 2 |
| AUC_{0-inf}, h·ng/m L | 2467. 8 | 537. 4 | 21. 8 | 1825. 8 | 2299. 7 | 3717. 9 | 2417. 4 | 416. 8 | 17. 2 | 1931. 4 | 2333. 4 | 3089. 6 |
| t_{1/2}, min | 106.1 | 14.7 | 13. 8 | 75.1 | 111.5 | 125.5 | 103.2 | 15.8 | 15. 3 | 66.2 | 107.8 | 101.9 |

**Table 64. In vitro release data of carbidopa-levodopa granules and carbidopa-levodopa tablets**

| | **Levodopa dissolution** | | | | **Carbidopa dissolution** | | | |
|---|---|---|---|---|---|---|---|---|
| **Sampling point/ min** | **Carbidopa-levodopa granules (25 mg/100 mg)** | **SD** | **Carbidopa-levodopa tablets (25 mg/100 mg)** | **SD** | **Carbidopa-levodopa granules (25 mg/100 mg)** | **SD** | **Carbidopa-levodopa tablets (25 mg/100 mg)** | **SD** |
| 5 | 92.9% | 8.70% | 77.5% | 15.49 % | 91.5% | 8.58 % | 78.3% | 14.76 % |
| 10 | 98.5% | 2.55% | 100.1% | 2.88 % | 97.3% | 2.42 % | 98.7% | 2.95 % |
| 15 | 99.2% | 1.51% | 101.7% | 1.07 % | 97.9% | 1.47 % | 100.0% | 1.26 % |
| 20 | 99.5% | 1.73% | 101.8% | 0.88 % | 98.2% | 1.74 % | 100.0% | 1.48 % |
| 30 | 99.4% | 1.40% | 102.3% | 0.87 % | 98.0% | 1.38 % | 100.8% | 1.17 % |

### Effect Example 5

The voriconazole granules obtained in Example 35, Example 45, and Comparative Example 4 were tested for dissolution and related substances, and the results are shown in Table 65. The dissolution method used was USP Method 2 (paddle method) at a rotation speed of 50 rpm. The dissolution medium was 0.1N hydrochloric acid at pH 1.0, with a volume of 900 mL. Samples were taken at 5, 10, 20, 30, 45, and 60 minutes for testing.

Comparing Example 35, Example 45, and Comparative Example 4, when glyceryl behenate 888 ATO was used as the hot-melt adhesive, the dissolution of voriconazole granules in the first 20 minutes was significantly slower than that of granules using Poloxamer 407 or polyethylene glycol 6000 as the adhesive. When polyethylene glycol 6000 was used as the adhesive, the dissolution of voriconazole granules showed no significant difference compared to granules using Poloxamer 407 as the adhesive. There was no significant difference in the related substances of voriconazole granules prepared using the same proportion of the above three hot-melt adhesives.

**Table 65. Dissolution and related substance test results of Example 35, Example 45, and Comparative Example 4**

| Test Item | Acceptance criteria | | Example 35 | Example 45 | Comparative Example 4 |
|---|---|---|---|---|---|
| Dissolution | Time min | | Voriconazole release% | | |
| | 5 | | 79.9 | 81.9 | 38.0 |
| | 10 | | 96.0 | 96.3 | 64.2 |
| | 20 | | 99.2 | 97.5 | 89.2 |
| | 30 | | 99.6 | 98.0 | 96.7 |
| | 45 | | 99.7 | 98.3 | 98.8 |
| | 60 | | 100.1 | 97.9 | 98.8 |
| Related substances | Impurity C | ≤ 0.2 | 0.005 | 0.004 | 0.004 |
| | Maximum single unknown impurity% | ≤ 0.2 | 0.01 | 0.01 | 0.01 |
| | Total impurities% | ≤ 1.0 | 0.03 | 0.02 | 0.02 |

The voriconazole granules obtained in Examples 48, 50, and 52 were tested for dissolution, and the results are shown in Table 66. The dissolution method used was USP Method 2 (paddle method) at a rotation speed of 50 rpm. The dissolution medium was 0.1N hydrochloric acid at pH 1.0, with a volume of 900 mL. Samples were taken at 5, 10, 20, 30, 45, and 60 minutes for testing.

**Table 66. Dissolution test results of Example 48, Example 50, and Example 52**

| Test Item | Acceptance criteria | Example 48 | Example 50 | Example 52 |
|---|---|---|---|---|
| Dissolution | Time min | Voriconazole release% | | |
| | 5 | 88.3 | 84.5 | 89.0 |
| | 10 | 93.4 | 95.3 | 95.1 |
| | 20 | 94.3 | 97.9 | 96.9 |
| | 30 | 94.5 | 98.1 | 97.6 |
| | 45 | 94.4 | 98.2 | 97.8 |
| | 60 | 94.7 | 98.2 | 97.5 |

### Effect Example 6

For the formulation of voriconazole granules in Example 31, compatibility studies of the raw materials and excipients under conditions of 40°C/75% RH revealed, as shown in Table 67, that among all excipients, Poloxamer 407 exhibited poor compatibility with voriconazole. After preparation into the formulation, there was a risk of exceeding the limit for related substances in terms of stability.

The oral administration delivery formulation kit containing voriconazole obtained in Example 44 was placed under conditions of 40°C/75% RH. Samples were taken and tested at 1 month, 3 months, and 6 months, with dissolution and related substances as the test indicators. The results are shown in Table 68. The dissolution method used was USP Method 2 (paddle method) at a rotation speed of 50 rpm. The dissolution medium was 0.1N hydrochloric acid at pH 1.0, with a volume of 900 mL. Samples were taken at 5, 10, 20, 30, 45, and 60 minutes for testing. After 6 months of accelerated testing, the dissolution of voriconazole granules did not show significant changes, and related substances were far below the limits. This indicates that the use of hot-melt granulation process and moisture-proof packaging can significantly improve the compatibility of voriconazole with Poloxamer 407 and increase the stability of the granules. Compared to the original drug (voriconazole dry suspension), which required storage at 2-8°C, the oral administration delivery formulation kit containing voriconazole in this effect example could be stored long-term under ambient temperature conditions, showing clear advantages in improving stability and storage conditions.

**Table 67. Results of compatibility study on voriconazole granule raw material**

| Sample (40°C/75%RH) | Total impurities% | | | Appearance |
|---|---|---|---|---|
| | 0 Days | 14 Days | 28 Days | |
| Voriconazole | 0.01 | 0.01 | 0.01 | No change |
| Voriconazole: Mannitol (1:10) | 0.01 | 0.02 | 0.02 | No change |
| Voriconazole: Poloxamer 407 (1:5) | 0.01 | 20.27 | 22.16 | Changed from a white powder to a yellow block |
| Voriconazole: Polyethylene glycol 6000 (1:5) | 0.01 | 0.45 | 0.63 | Changed from a white powder to a pale yellow liquid |
| Voriconazole: Low-substituted hydroxypropyl cellulose (1:5) | 0.01 | 0.02 | 0.03 | No change |
| Voriconazole: Colloidal silica (1:5) | 0.01 | 0.03 | 0.04 | No change |

**Table 68. Stability test results of Example 44**

| Test Item | Acceptance criteria | | 0 Days | 1 Month | 3 Months | 6 Months |
|---|---|---|---|---|---|---|
| Dissolution | Time min | | Voriconazole release% | | | |
| | 5 | | 83.0 | 84.6 | 88.1 | 86.1 |
| | 10 | | 96.2 | 94.5 | 97.4 | 96.4 |
| | 20 | | 96.6 | 94.9 | 97.6 | 96.7 |
| | 30 | | 97.1 | 95.0 | 97.8 | 96.8 |
| | 45 | | 97.1 | 95.2 | 97.9 | 96.9 |
| | 60 | | 97.3 | 95.3 | 97.8 | 97.0 |
| Related substances | Impurity C | ≤ 0.2 | 0.01 | 0.05 | 0.06 | 0.04 |
| | Maximum single unknown impurity% | ≤ 0.2 | Not detected | Not detected | Not detected | Not detected |
| | Total impurities% | ≤ 1.0 | 0.01 | 0.05 | 0.06 | 0.04 |

### Effect Example 7

The oral administration delivery formulation kit containing voriconazole obtained in Example 44 was compared with a commercially available voriconazole dry suspension. The preference statistics for 10 participants regarding the two products in terms of preparation before use, mode of use, storage conditions, appearance, and comprehensive factors are as follows:

**Table 69. Preference statistical results of the oral administration delivery formulation kit of the present disclosure and the commercially available voriconazole dry suspension**

| Name | Oral administration delivery formulation kit of Example 44 | Commercially available dry suspension | Both are acceptable |
|---|---|---|---|
| Preparation before use | 8 | 0 | 2 |
| Mode of use | 8 | 0 | 2 |
| Storage conditions | 10 | 0 | 0 |
| Appearance | 9 | 0 | 1 |
| Comprehensive factors | 9 | 0 | 1 |

(The values in the table represent the number of participants who preferred the example or the commercially available product. For instance, when considering "preparation before use", 8 participants preferred the oral administration delivery formulation kit of Example 44, no participants preferred the commercially available dry suspension, and 2 participants considered both products acceptable.)

It can be concluded that the majority of the surveyed participants considered the oral administration delivery formulation kit containing voriconazole of the present disclosure to be more convenient for use and storage, more attractive in appearance, and preferred to choose the product of the present disclosure; only a small number of participants considered both products acceptable in terms of preparation before use, mode of use, and appearance; overall, the vast majority believed that the oral administration delivery formulation kit containing voriconazole of the present disclosure has greater advantages compared to the commercially available dry suspension.

## Claims

1. A pharmaceutical composition comprising the following components: an active pharmaceutical ingredient and a hot-melt adhesive; wherein
the mass percentage of the active pharmaceutical ingredient is 0.3%-63%;
the hot-melt adhesive is:
Class A hot-melt adhesive: Poloxamer P188 and/or Poloxamer P407;
or Class B hot-melt adhesive: polyethylene glycol 6000;
the mass percentage of the Class A hot-melt adhesive in the pharmaceutical composition is 5%-30%;
the mass percentage of the Class B hot-melt adhesive in the pharmaceutical composition is 12%-25%;
the pharmaceutical composition is a pharmaceutical composition in the form of a hot-melt granulated granule.

2. The pharmaceutical composition according to claim 1, wherein the mass percentage of the Class A hot-melt adhesive in the pharmaceutical composition may be 14%-25%; and the mass percentage of the Class B hot-melt adhesive in the pharmaceutical composition may be 15.0%-21.6%.

3. A pharmaceutical composition comprising the following components: an active pharmaceutical ingredient and a hot-melt adhesive; wherein
the mass percentage of the active pharmaceutical ingredient is 0.3%-50%;
the hot-melt adhesive is Poloxamer P188 and/or Poloxamer P407;
the mass percentage of the hot-melt adhesive in the pharmaceutical composition is 5%-30%.

4. The pharmaceutical composition according to any one of claims 1-3, wherein the active pharmaceutical ingredient comprises, but is not limited to, one or more of the following: dopaminergic agents, anti-erectile dysfunction agents, antiviral agents, gastric acid secretion inhibitors, anticoagulants, antifungal agents, antibacterial agents, antiasthmatic agents, antidepressants, antiepileptic agents, antiallergic agents, antihypertensive agents, antipsychotic agents, analgesic agents, anti-inflammatory agents, antiemetic agents, and antitussive agents; preferably, the active pharmaceutical ingredient is a poorly soluble and easily degradable drug, a poorly soluble and non-easily degradable drug, a non-poorly soluble and easily degradable drug, or a non-poorly soluble and non-easily degradable drug, wherein preferably,
the poorly soluble and easily degradable drug is selected from: ritonavir, vonoprazan, rivaroxaban, posaconazole, cefdinir, cefixime, cefuroxime axetil, clarithromycin, linezolid, and duloxetine;
the poorly soluble and non-easily degradable drug is selected from: apixaban, tadalafil, dapoxetine, contezolid, olanzapine, aripiprazole, quetiapine fumarate, zolmitriptan, rizatriptan, almotriptan, naratriptan, frovatriptan, eletriptan, and verapamil;
the non-poorly soluble and easily degradable drug is selected from: carbidopa, oseltamivir phosphate, favipiravir, molnupiravir, deuremidevir hydrobromide, nirmatrelvir, cefaclor, cefprozil, amoxicillin, montelukast sodium, levetiracetam, and desloratadine;
the non-poorly soluble and non-easily degradable drug is selected from: levodopa, sildenafil, vardenafil, tegoprazan, fluconazole, escitalopram oxalate, levocetirizine, amlodipine, ketorolac tromethamine, and diphenhydramine.

5. The pharmaceutical composition according to any one of claims 1-4, wherein the pharmaceutical composition meets one or more of the following conditions:
the mass percentage of the active pharmaceutical ingredient in the pharmaceutical composition is 0.3%-40%;
the mass percentage of the hot-melt adhesive in the pharmaceutical composition is 14%-25%;
the pharmaceutical composition is a pharmaceutical composition in the form of a hot-melt granulated granule;
the pharmaceutical composition does not comprise an effervescent system;
the pharmaceutical composition further comprises one or more of the following components: a disintegrant, a filler, a lubricant, a glidant, a flavoring agent, and an aromatic agent.

6. The pharmaceutical composition according to any one of claims 1-5, wherein the pharmaceutical composition meets one or more of the following conditions:
the disintegrant is one or more of croscarmellose sodium, low-substituted hydroxypropyl cellulose, sodium starch glycolate, and crospovidone, preferably croscarmellose sodium and/or low-substituted hydroxypropyl cellulose;
the mass percentage of the disintegrant in the pharmaceutical composition is 0%-30%, preferably 10%-22%;
the filler is mannitol and/or lactose;
the mass percentage of the filler in the pharmaceutical composition is 0%-50%;
the lubricant is one or more of calcium stearate, glyceryl behenate, magnesium stearate, sodium stearyl fumarate, magnesium silicate, and calcium silicate;
the mass percentage of the lubricant in the pharmaceutical composition is 0%-2%;
the glidant is talc and/or colloidal silica;
the mass percentage of the glidant in the pharmaceutical composition is 0%-2%;
the flavoring agent is one or more of sucrose, maltose, stevioside, mannitol, erythrose, and aspartame, preferably one or more of stevioside, mannitol, erythrose, and aspartame;
the mass percentage of the flavoring agent in the pharmaceutical composition is 0%-5%;
the aromatic agent is a flavor, preferably a natural flavor;
the mass percentage of the aromatic agent in the pharmaceutical composition is 0%-3%.

7. The pharmaceutical composition according to claim 5 or 6, wherein the pharmaceutical composition meets one or more of the following conditions:
the poorly soluble and non-easily degradable drug is also voriconazole;
the pharmaceutical composition further comprises a surfactant; preferably, the surfactant is one or more of glyceryl monostearate, soy lecithin, lecithin, xanthan gum, polyoxyethylene stearate, and sodium dodecyl sulfate; preferably, the mass percentage of the surfactant in the pharmaceutical composition is 0%-15%;
the disintegrant is also microcrystalline cellulose and/or pregelatinized starch; the mass percentage of the disintegrant in the pharmaceutical composition is further 0%-22%;
the filler is also sorbitol;
the flavoring agent is also citric acid.

8. The pharmaceutical composition according to any one of claims 1-7, wherein the active pharmaceutical ingredient is carbidopa and levodopa; the mass percentage of carbidopa in the pharmaceutical composition is 0.5%-15%, preferably 2%-15%; the mass percentage of levodopa in the pharmaceutical composition is 2%-48%, preferably 5%-48%; the mass ratio of carbidopa to levodopa is 1:1-1:24;
preferably, the pharmaceutical composition comprises the following components in percentage by mass: 0.5%-10% carbidopa, 2%-40% levodopa, 6%-30% hot-melt adhesive, and 0-30% disintegrant; or, the pharmaceutical composition comprises the following components in percentage by mass: 0.5%-10% carbidopa, 2%-40% levodopa, 6%-30% Class A hot-melt adhesive, and 0-30% disintegrant;
more preferably, the pharmaceutical composition comprises the following components in percentage by mass: 5.7%-8.3% carbidopa, 23%-33% levodopa, 15%-25% hot-melt adhesive, 10.1%-20.1% low-substituted hydroxypropyl cellulose, and 23.2%-33.2% mannitol; or, the pharmaceutical composition comprises 6.5%-7.5% carbidopa, 25.1%-30.1% levodopa, 17.5%-22.5% hot-melt adhesive, 13.5%-18.5% low-substituted hydroxypropyl cellulose, and 27.0%-32.0% mannitol.

9. A pharmaceutical composition comprising an active pharmaceutical ingredient and a hot-melt adhesive; wherein the active pharmaceutical ingredient is voriconazole, and the hot-melt adhesive is:
Class A hot-melt adhesive: Poloxamer P188 and/or Poloxamer P407;
or Class B hot-melt adhesive: polyethylene glycol 6000;
the mass percentage of the Class B hot-melt adhesive in the pharmaceutical composition is 12%-25%;
the pharmaceutical composition is a pharmaceutical composition in the form of a hot-melt granulated granule.

10. A pharmaceutical composition comprising an active pharmaceutical ingredient and a hot-melt adhesive; wherein the active pharmaceutical ingredient is voriconazole, and the hot-melt adhesive is selected from:
Class A hot-melt adhesive: Poloxamer P188 and/or Poloxamer P407;
and Class B hot-melt adhesive: polyethylene glycol 6000;
the mass percentage of the Class B hot-melt adhesive in the pharmaceutical composition is 12%-25%;
the pharmaceutical composition is a pharmaceutical composition in the form of a hot-melt granulated granule.

11. The pharmaceutical composition according to claim 9 or 10, wherein the mass percentage of the active pharmaceutical ingredient is 1%-63%;
the mass percentage of the Class A hot-melt adhesive in the pharmaceutical composition is 5%-30%.

12. The pharmaceutical composition according to any one of claims 9-11, wherein the pharmaceutical composition meets one or more of the following conditions:
the mass percentage of the active pharmaceutical ingredient in the pharmaceutical composition is 8.3%-63%;
the mass percentage of the Class A hot-melt adhesive in the pharmaceutical composition is 12%-25%, preferably 15.0%-21.6%;
the mass percentage of the Class B hot-melt adhesive in the pharmaceutical composition is preferably 15.0%-21.6%;
the pharmaceutical composition further comprises one or more of the following components: a disintegrant, a filler, a lubricant, a glidant, and a flavoring agent.

13. The pharmaceutical composition according to any one of claims 9-12, wherein the pharmaceutical composition meets one or more of the following conditions:
the disintegrant is one or more of croscarmellose sodium, low-substituted hydroxypropyl cellulose, sodium starch glycolate, microcrystalline cellulose, pregelatinized starch, and crospovidone, preferably croscarmellose sodium and/or low-substituted hydroxypropyl cellulose;
the mass percentage of the disintegrant in the pharmaceutical composition is 0%-30%, preferably 0%-20%;
the filler is one or more of mannitol, sorbitol, and lactose;
the mass percentage of the filler in the pharmaceutical composition is 0%-50%;
the lubricant is one or more of calcium stearate, glyceryl behenate, magnesium stearate, sodium stearyl fumarate, magnesium silicate, and calcium silicate;
the mass percentage of the lubricant in the pharmaceutical composition is 0%-2%;
the glidant is talc and/or colloidal silica;
the mass percentage of the glidant in the pharmaceutical composition is 0%-2%;
the flavoring agent is one or more of sucrose, maltose, stevioside, mannitol, erythrose, aspartame, citric acid, and flavor, preferably one or more of stevioside, mannitol, erythrose, aspartame, and flavor;
the mass percentage of the flavoring agent in the pharmaceutical composition is 0%-5%.

14. The pharmaceutical composition according to any one of claims 9-13, wherein the pharmaceutical composition further comprises a surfactant; preferably, the surfactant is one or more of glyceryl monostearate, soy lecithin, lecithin, xanthan gum, polyoxyethylene stearate, and sodium dodecyl sulfate; the mass percentage of the surfactant in the pharmaceutical composition is 0%-15%.

15. The pharmaceutical composition according to any one of claims 9-14, wherein the pharmaceutical composition comprises the following components in percentage by mass: 1%-63% of the active pharmaceutical ingredient, "5%-30% of the Class A hot-melt adhesive or 12%-25% of the Class B hot-melt adhesive", 0%-30% of the disintegrant, 0%-50% of the filler, 0%-2% of the lubricant, 0%-2% of the glidant, and 0%-5% of the flavoring agent;
or, the pharmaceutical composition comprises the following components in percentage by mass: 1%-63% of the active pharmaceutical ingredient, 5%-30% of "the Class A hot-melt adhesive and the Class B hot-melt adhesive", 0%-30% of the disintegrant, 0%-50% of the filler, 0%-2% of the lubricant, 0%-2% of the glidant, and 0%-5% of the flavoring agent;
preferably, the pharmaceutical composition comprises the following components in percentage by mass: 8.3%-63% of voriconazole, 12%-25% of the hot-melt adhesive, 0%-20% of the disintegrant, 0%-50% of the filler, 0%-2% of the lubricant, 0%-2% of the glidant, and 0%-5% of the flavoring agent;
more preferably, the pharmaceutical composition comprises the following components in percentage by mass: 33%-55% of voriconazole, 15.0%-21.6% of the hot-melt adhesive, 5%-18% of the disintegrant, 8.5%-36.5% of the filler, 0%-1% of the lubricant, 0.5%-1.5% of the glidant, and 0.5%-1.5% of the flavoring agent.

16. A pharmaceutical formulation, wherein the pharmaceutical formulation is a hot-melt granulated granule, and the pharmaceutical formulation comprises the pharmaceutical composition according to any one of claims 1-8.

17. An oral administration delivery formulation kit, wherein the oral administration delivery formulation kit comprises an oral administration delivery device AcuSiS^{®} and the pharmaceutical formulation according to claim 16; the pharmaceutical formulation is disposed in a drug-carrying space of the oral administration delivery device AcuSiS^{®};
preferably, the filling amount of the pharmaceutical composition is 50-1000 mg.

18. A pharmaceutical formulation, wherein the pharmaceutical formulation is a hot-melt granulated granule, and the pharmaceutical formulation comprises the pharmaceutical composition according to any one of claims 9-15; preferably, the particle size of the hot-melt granulated granule is less than 1000 µm; preferably, the pharmaceutical formulation is a carbidopa-levodopa granule comprising 25 mg of carbidopa, 100 mg of levodopa, 66.9 mg of a hot-melt adhesive, 54.6 mg of low-substituted hydroxypropyl cellulose, and 113.3 mg of mannitol.

19. An oral administration delivery formulation kit, wherein the oral administration delivery formulation kit comprises an oral administration delivery device AcuSiS^{®} and the pharmaceutical formulation according to claim 18; the pharmaceutical formulation is disposed in a drug-carrying space of the oral administration delivery device AcuSiS^{®};
preferably, the filling amount of the pharmaceutical formulation is 50-1000 mg;
preferably, the pharmaceutical formulation is a carbidopa-levodopa granule comprising 25 mg of carbidopa, 100 mg of levodopa, 66.9 mg of a hot-melt adhesive, 54.6 mg of low-substituted hydroxypropyl cellulose, and 113.3 mg of mannitol.

20. The oral administration delivery formulation kit according to claim 17 or 19, wherein each said oral administration delivery formulation kit further comprises an encapsulation layer for sealing the oral administration delivery device AcuSiS^{®};
preferably, the encapsulation layer is a polyester-aluminum-polyethylene composite film bag;
preferably, a desiccant is further provided in the encapsulation layer, wherein the desiccant is a bagged desiccant, more preferably a 0.25 g bagged desiccant.

21. A use of the pharmaceutical composition according to claim 8 in the manufacture of a medicament for emergency or intermittent treatment of a Parkinson's off episode.

22. A use of the pharmaceutical composition according to any one of claims 9-15 in the manufacture of a medicament for the treatment or prevention of invasive fungal disease.

23. A preparation method for the pharmaceutical formulation according to claim 16 or 18, wherein the preparation method is a hot-melt granulation method, comprising the following steps:
pulverizing the hot-melt adhesive to obtain a powder, mixing with the active pharmaceutical ingredient, a disintegrant, and a filler, performing hot-melt granulation, then uniformly mixing with the remaining components of the pharmaceutical composition, and sieving to obtain the pharmaceutical formulation;
alternatively, pulverizing the hot-melt adhesive to obtain a powder, mixing with the active pharmaceutical ingredient, a disintegrant, a filler, a lubricant, and a glidant, performing hot-melt granulation, then uniformly mixing with the remaining components of the pharmaceutical composition, and sizing and sieving to obtain the pharmaceutical formulation.
